Europäisches Patentamt

European Patent Office

Office européen des brevets

(19)

(11) **EP 1 216 660 A2**

(12) **EUROPÄISCHE PATENTANMELDUNG**

(43) Veröffentlichungstag:
    **26.06.2002 Patentblatt 2002/26**

(51) Int Cl.$^7$: **A61B 6/03**

(21) Anmeldenummer: **01000768.0**

(22) Anmeldetag: **18.12.2001**

(84) Benannte Vertragsstaaten:
**AT BE CH CY DE DK ES FI FR GB GR IE IT LI LU MC NL PT SE TR**
Benannte Erstreckungsstaaten:
**AL LT LV MK RO SI**

(30) Priorität: **20.12.2000 DE 10063442**

(71) Anmelder:
- **Philips Corporate Intellectual Property GmbH
  52066 Aachen (DE)**

- **Koninklijke Philips Electronics N.V.
  5621 BA Eindhoven (NL)**

(72) Erfinder: **Schomberg, Hermann
  52066, Aachen (DE)**

(74) Vertreter: **Volmer, Georg, Dipl.-Ing. et al
  Philips Corporate
  Intellectual Property GmbH
  Weisshausstr. 2
  52066 Aachen (DE)**

(54) **Verfahren und Röntgeneinrichtung zur Ermittlung eines Satzes von Projektionsabbildungen eines Untersuchungsobjektes**

(57) Die Erfindung betrifft ein Verfahren zur Ermittlung eines Satzes von Projektionsabbildungen für die Rekonstruktion eines dreidimensionalen Bilddatensatzes eines in einem Untersuchungsbereich angeordneten Untersuchungsobjektes mittels einer eine Röntgenquelle und einen Röntgendetektor aufweisenden Röntgeneinrichtung, wobei die Röntgenquelle zur Erfassung der Projektionsabbildungen entlang einer im Wesentlichen auf einer Kugeloberfläche liegenden Trajektorie um den Untersuchungsbereich geführt wird. Erfindungsgemäß ist vorgesehen, dass die Trajektorie derart ausgestaltet ist, dass die Röntgenquelle zur Ermittlung des Satzes von Projektionsabbildungen die Trajektorie kontinuierlich durchlaufen kann und dass nicht alle Punkte der Trajektorie in einer gemeinsamen Ebene liegen. Dadurch wird es möglich, auf schnelle Weise einen Projektionsdatensatz zu gewinnen, der eine genaue Rekonstruktion des Untersuchungsgegenstandes ermöglicht. Die Erfindung betrifft außerdem eine entsprechende Röntgeneinrichtung.

FIG.10a

EP 1 216 660 A2

## Beschreibung

Die Erfindung betrifft ein Verfahren zur Ermittlung eines Satzes von Projektionsabbildungen für die Rekonstruktion eines dreidimensionalen Bilddatensatzes eines in einem Untersuchungsbereich angeordneten Untersuchungsobjektes mittels einer eine Röntgenquelle und einen Röntgendetektor aufweisenden Röntgeneinrichtung, wobei die Röntgenquelle zur Erfassung der Projektionsabbildungen entlang einer im Wesentlichen auf einer Kugeloberfläche liegenden Trajektorie um den Untersuchungsbereich geführt wird. Außerdem betrifft die Erfindung auch eine entsprechende Röntgeneinrichtung.

Bei der so genannten Kegelstrahlcomputertomographie wird versucht, ein dreidimensionales Bild eines Untersuchungsobjektes aus einem Satz von Kegelstrahlprojektionen dieses Objektes zu rekonstruieren. Zur Messung der Kegelstrahlprojektionen wird eine Untersuchungseinrichtung benutzt, die mit einer punktförmigen Röntgenquelle und einem flächigen Röntgendetektor versehen ist. Das Objekt befindet sich zwischen Quelle und Detektor. Während das Objekt stationär bleibt, werden Quelle und Detektor um das Objekt herumgeführt, wobei in kurzen räumlichen bzw. zeitlichen Abständen Kegelstrahlprojektionen gemessen werden. In der Regel werden Quelle und Detektor starr miteinander gekoppelt, und die Verbindungslinie zwischen der Quelle und dem Mittelpunkt des Detektors geht stets durch einen ausgezeichneten Punkt, das Isozentrum. In diesem Fall bestimmt die Trajektorie der Quelle auch die Trajektorie des Detektors. Außerdem liegt die Trajektorie der Quelle, von kleinen mechanischen Ungenauigkeiten abgesehen, auf der Oberfläche einer Kugel, deren Mittelpunkt das Isozentrum ist. Die Trajektorie der Quelle kann durch eine Abbildung $\mathbf{a} :[s_-,s_+]\to \mathbf{R}^3$ beschrieben werden, wobei s ein reeller Parameter ist und $\mathbf{a}(s)$ den Ortsvektor der Trajektorie bezüglich eines kartesischen Koordinatensystems bezeichnet, dessen Mittelpunkt im Isozentrum liegt. Das rekonstruierte Bild des Objektes gibt die räumliche Verteilung des Röntgenschwächungskoeffizienten im.

Untersuchungsbereich wieder. Das Bild wird mit Hilfe eines Computers und eines Rekonstruktionsalgorithmus aus dem gemessenen Satz von Kegelstrahlprojektionen Damit eine genaue Rekonstruktion des Röntgenschwächungskoeffizienten möglich wird, müssen zahlreiche Voraussetzungen erfüllt sein. Eine dieser Voraussetzungen wird z. B. von P. Grangeat in "Mathematical framework of cone beam 3D reconstruction via the first derivative of the Radon transform", in G. T. Herman, A K. Louis und F. Natterer, Mathematical Methods in Tomography, Band 1497 der Lecture Notes in Mathematics, Springer Verlag, 1991, auf den Seiten 66-97 angsgsben und begründet. Diese als Vollständigkeitsbedingung bekannte Voraussetzung besagt, dass jede Ebene, die den Untersuchungsbereich schneidet, auch die Trajektorie der Röntgenquelle schneiden muss. Im Folgenden wird eine Trajektorie, die die Vollständigkeitsbedingung bezüglich eines Untersuchungsbereiches erfüllt, als vollständig bezüglich dieses Untersuchungsbereiches bezeichnet.

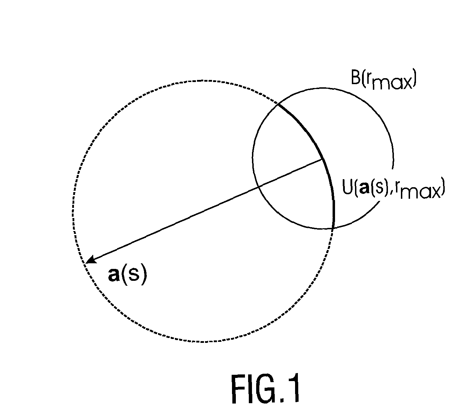

Bei einer isozentrischen Untersuchungseinrichtung ist der Untersuchungsbereich vorzugsweise eine isozentrische Kugel $B(r_{max})$ mit dem Radius $r_{max}$. Für einen kugelförmigen Untersuchungsbereich lässt sich die Vollständigkeitsbedingung auch anders formulieren. Zur Herleitung dieser alternativen Formulierung betrachtet man zunächst die Menge aller derjenigen Ebenen, die einen beliebigen aber festen Punkt $\mathbf{a}(s)$ der Trajektorie sowie die Kugel $B(r_{max})$ schneiden. Jede dieser Ebenen ist eindeutig durch ihren Normalenvektor in Bezug auf den Mittelpunkt der Kugel $B(r_{max})$, also das Isozentrum, charakterisiert. Einfache geometrische Überlegungen, die anhand von Fig. 1 nachvollzogen werden können, zeigen nun, dass diese Normalenvektoren eine Kugelkappe $U(\mathbf{a}(s),r_{max})$ bilden, wobei die zugehörige Kugel den Mittelpunkt $\mathbf{a}(s)/2$ und den Radius $\mathbf{a}(s)/2$ |hat. Variiert man den Parameter s und damit den Punkt $\mathbf{a}(s)$, so variiert auch die Kugelkappe $U(\mathbf{a}(s),r_{max})$. Wenn der Parameter s das Intervall $[s_-,s_+]$ durchläuft, so erhält man eine entsprechende Menge von Kugelkappen. Diese Menge von Kugelkappen enthält konstruktionsgemäß genau diejenigen Normalenvektoren, die zu denjenigen Ebenen gehören, die sowohl die Trajektorie als auch den kugelförmigen Untersuchungsbereich $B(r_{max})$ schneiden. Die Erfüllung der Vollständigkeitsbedingung verlangt also, dass die Menge dieser Kugelkappen die Kugel $B(r_{max})$ vollständig ausfüllt. Wäre nämlich eine Lücke vorhanden, so würden die Ebenen, die zu den Normalenvektoren in dieser Lücke gehören, zwar den Untersuchungsbereich schneiden, nicht aber die Trajektorie.

Bei gegebener Trajektorie und gegebener Kugel $B(r_{max})$ kann man mit Hilfe eines Computers und eines geeigneten Computerprogrammes eine dichte Teilmengs der Menge aller Kugelkappen berechnen und graphisch darstellen und dann visuell überprüfen, ob diese Kugelkappen die Kugel $B(r_{max})$ lückenlos ausfüllen oder nicht. Im Gegensatz zur ersten Formulierung der Vollständigkeitsbedingung erlaubt die zweite Formulierung also einen anschaulichen Test, ob eine gegebene Trajektorie bezüglich einer gegebenen Kugel $B(r_{max})$ vollständig ist.

Zu beachten ist, dass eine planare Trajektorie, also eine Trajektorie, die ganz in einer Ebene liegt, nicht vollständig sein kann. Alle Ebenen, die zur Ebene der Trajektorie parallel und von ihr verschieden sind, schneiden die Trajektorie nämlich nicht. Insbesondere kann also eine kreisförmige Trajektorie oder ein Teilstück davon nicht vollständig sein. Es gibt aber aus planaren Teilstücken zusammengssetzte Trajektorien, die vollständig sind Dazu zählen z. B. zwei Kreise, die denselben Durchmesser und Mittelpunkt haben und deren Achsen einen hinreichend großen Winkel miteinander bilden.

Wenn die Trajektorie der Röntgenquelle nicht vollständig ist, kann trotzdem versucht werden, ein Bild des Untersuchungsobjektes zu rekonstruieren. Im allgemeinen müssen dann aber Abstriche bei der Bildqualität hingsnommen werden.

Die Trajektorie der Röntgenquelle muss aber auch durch die Untersuchungseinrichtung realisierbar sein. In medizinischen Anwendungen ist das Untersuchungsobjekt ein Teil eines Patienten, der auf einem Untersuchungstisch liegt, und es ist dafür zu sorgen, dass Röntgenquelle und Röntgendetektor nicht gegen das Untersuchungsobjekt oder seine Unterlage stoßen.

Eine Untersuchungseinrichtung nach dem Stand der Technik ist das Philips INTEGRIS V5000. Diese Untersuchungseinrichtung besitzt einen C-Arm, an dessen einem Ende eine Röntgenquelle und an dessen anderem Ende ein Röntgendetektor befestigt ist. Zwischen der Röntgenquelle und dem Röntgendetektor wird das Untersuchungsobjekt angeordnet. Der C-Arm wird von einer kreisförmig gebogenen Schiene gehalten, so dass er um eine Achse gedreht werden kann. Diese so genannte C-Arm Achse steht senkrecht auf der Ebene, die den C-Bogen enthält. Die Halterung für den C-Arm ist über ein Drehgelenk mit einem so genannten L-Arm verbunden, welcher seinerseits über ein Drehgelenk mit einer an der Decke befindlichen Aufhängevorrichtung verbunden ist. Diese Aufhängevorrichtung kann geradlinig und waagerecht verschoben werden. Die drei erwähnten Achsen schneiden sich stets in einem Punkt, dem Isozentrum. Ein Elektromotor sorgt für die steuerbare Rotation der Röntgenquelle und des Röntgendetektors um die C-Arm Achse. Drehungen um die beiden anderen Achsen werden zwar von Servomotoren unterstützt, sind aber nicht steuerbar. Die Aufnahme eines Satzes von Kegelstrahlprojektionen des Untersuchungsobjekts geschieht während einer Drehung des C-Arms um die C-Arm Achse. Drehungen um die beiden anderen Achsen sind wegen der fehlenden Steuermöglichkeit beim INTEGRIS V5000 während der Aufnahme von Kegelstrahlprojektionen nicht möglich. Die Rotation des C-Arms um seine C-Arm Achse führt zu einer halbkreisförmigen Trajektorie der Röntgenquelle. Wie bereits erwähnt, ist eine solche Trajektorie nicht vollständig.

Grundsätzlich könnte eine vollständige Trajektorie aus mehreren Halbkreisen zusammengesetzt werden Der C-Arm würde dann zwischen den Teiluntersuchungen neu positioniert werden. Dies ist aber mit großem zeitlichen Aufwand verbunden. Bei Anwendungen, welche zur Darstellung von Blutgefäßen die Verabreichung von Röntgenkontrastmitteln erfordern, würde auch die Menge des zu verabreichenden Kontrastmittels erhöht werden.

Es ist Aufgabe der vorliegenden Erfindung, ein geeigneteres Verfahren zur Gewinnung eines vollständigen Satzes von Kegelstrahlprojektionen eines in einem Untersuchungsbereich angeordneten Untersuchungsobjekts bereitzustellen. Es ist ferner Aufgabe der vorliegenden Erfindung, eine entsprechende Röntgeneinrichtung anzugsben.

Diese Aufgabe wird erfindungsgemäß durch ein Verfahren nach Anspruch 1 bzw. durch eine Röntgeneinrichtung gemäß Anspruch 11 gelöst. Erfindungsgemäß wird vorgeschlagen, dass die Trajektorie derart ausgestaltet ist, dass die Röntgenquelle zur Ermittlung des Satzes von Projektionsabbildungen die Trajektorie kontinuierlich durchlaufen kann und dass nicht alle Punkte der Trajektorie in einer gemeinsamen Ebene liegen.

Gemäß der ersten Vollständigkeitsbedingung muss jede Ebene, die das Untersuchungsobjekt schneidet, einen Punkt der Trajektorie aufweisen. Befindet sich die Trajektorie vollständig in einer Ebene, die das darzustellende Objekt schneidet, so erfüllt diese Trajektorie die Vollständigkeitsbedingung nicht. Denn jede Ebene, die parallel zur Trajektorienebene ausgerichtet ist und das Untersuchungsobjekt schneidet, enthält keinen Punkt der Trajektorie. Deshalb muss jede Trajektorie, die die Vollständigkeitsbedingung erfüllt, eine dreidimensionale Kurve sein, d. h. sie darf nicht in einer Ebene liegen.

Zwar liegt die bekannte Trajektorie aus zwei orthogonalen Kreisen nicht in einer Ebene und erfüllt zugleich die Vollständigkeitsbedingung. Sie ist jedoch derart ausgestaltet, dass die Röntgenquelle zunächst um eine ersten Rotationsachse um 360° rotiert wird und danach um eine zweite Rotationsachse senkrecht zur ersten Rotationsachse um 360° rotiert wird. Deshalb muss die Röntgenquelle nach einer Rotation um die eiste Rotationsachse angehalten werden und hierauf um die zweite Rotationsachse rotiert werden. Die Trajokterie kann also nicht kontinuierlich durchlaufen werden. Gemäß der Erfindung kann die Röntgenquelle dagegen entlang einer dreidimensionalen Trajektorie geführt werden, ohne angehalten zu werden. Deshalb ermöglicht die erfindungsgemäße Trajektorie die Ermittlung eines vollständigen Satzes von Projektionsabbildungen für einen 3D-Bilddatensatz auf zuverlässige und schnelle Weise in einem Durchlauf. Dies trägt dazu bei, dass die Rekonstruktion von 3D-Bildern im Wesentlichen frei von Artefakten und Ungenauigkeiten ist.

Gemäß einer bevorzugten Ausgestaltung der vorliegenden Erfindung ist die Trajektorie derart ausgestaltet, dass jede den Untersuchungsbereich schneidende Ebene mindestens einen Punkt der Trajektorie aufweist. Damit ist die Vollständigkeitsbedingung für den Untersuchungsbereich erfüllt, und eine genaue Abbildung eines in dem Untersuchungsbereich angaordneten Untersuchungsobjektes wird ermöglicht.

Gemäß einer bevorzugten Ausführungsform der vorliegenden Erfindung stellt die Trajektorie eine geschlossene Kurve dar. Eine geschlossene Kurve ist dadurch gekennzeichnet, dass die Röntgenquelle nach einem endlichen Zeitintervall zu ihrem Ausgangspunkt zurückkehrt, wenn sie entlang der Trajektorie bewegt wird. Es ist somit möglich, die Röntgenquelle mehrfach entlang der Trajektorie um das Untersuchungsobjekt zu bewegen, während Projektionsabbildungen aufgenommen werden. Eine solche Möglichkeit ist von Vorteil zur Abbildung von sich periodisch bewegenden Organen, etwa des schlagenden Herzens.

Vorzugsweise stellt die erfindungsgemäße Trajektorie eine zweimal differenzierbare Kurve dar. Wählt man als Kurvenparameter die Zeit t, zu der die Röntgenquelle am Ort a(t) der Trajektorie ist, so ist die Geschwindigkeit der Röntgenquelle zu jedem beliebigen Zeitpunkt durch die erste Ableitung der Kurve nach der Zeit gegeben. Da die Kurve zweimal differenzierbar ist, existiert die erste Ableitung. Ferner ist die erste Ableitung stetig, da die Kurve zweimal differenzierbar ist. Der Ort der Röntgenquelle auf der Trajektorie verändert sich demnach stetig mit der Zeit. Deshalb kann eine solche Kurve von einer Röntgenquelle kontinuierlich durchfahren werden. Bei einer sprunghaften, also nicht stetigen Änderung des Orts der Röntgenquelle, müsste hingegen die Röntgenquelle an einer solchen Sprungstelle angehalten werden, um die Trajektorie zu durchfahren. Vorzugsweise wird eine erfindungsgemäße Trajektorie sogar als zweimal stetig differenzierbare Kurve, insbesondere mit großen Krümmungsradien gewählt. Auf diese Weise werden die bei der Realisierung der Trajektorie auftretenden Beschleunigungen klein gehalten.

Bevorzugt werden erfindungsgemäß Kegelstrahlprojektionen als Projektionsabbildungen erfasst.

Die erfindungsgemäße Röntgeneinrichtung ist derart ausgestaltet, dass die Röntgenquelle zur Ermittlung des Satzes von Projektionsabbildungen die Trajektorie kontinuierlich durchlaufen kann und dass nicht alle Punkte der Trajektorie in einer gemeinsamen Ebene liegen.

Vorzugsweise ist die erfindungsgemäße Röntgeneinrichtung ein C-Arm-System mit einem C-Arm, an dessen einem Ende die Röntgenquelle und an dessen anderem Ende der Röntgendetektor befestigt ist. Ein C-Arm erlaubt es, das Untersuchungsobjekt zwischen Quelle und Detektor zu platzieren. Wird die Röntgenquelle entlang der Trajektorie geführt, so vollzieht der Röntgendetektor eine entsprechende Bewegung. Die Bewegung der Röntgenquelle um das Untersuchungsobjekt kann auf einfache Weise durch Bewegen des C-Arms erfolgen.

Die Röntgeneinrichtung ist vorzugsweise derart ausgestaltet, dass der C-Arm um eine C-Arm Achse rotierbar ist, während gleichzeitig die C-Arm Halterung um eine Propellerachse drehbar ist. Die Propellerachse und die C-Arm Achse stehen senkrecht aufeinander und besitzen einen gemeinsamen Schnittpunkt, das sogenannte Isozentrum. Auch die gerade Verbindungslinie zwischen dem Brennfleck der Röntgenquelle und dem Mittelpunkt des Detektors geht durch das Isozentrum. Mit der vorstehenden Anordnung von Rotationsachsen wird bewirkt, dass die Röntgenquelle bei einer Rotation um eine der Rotationsachsen auf einer Kugeloberfläche um das Isozentrum rotiert wird. Das Untersuchungsobjekt wird zur Ermittlung eines 3D-Bilddatensatzes im Bereich des Isozentrums untergebracht. Die Propellerachse und der C-Arm liegen im Wesentlichen in einer gemeinsamen Ebene. Bei einer Rotation des C-Arms um die Propellerachse werden die Abschnitte des C-Arms, an dem die Röntgenquelle und der Röntgendetektor befestigt sind, wie die Blätter eines Propellers um eine gemeinsame Achse rotiert. Die Drehung um die Propellerachse kann dabei mehr als 360° bzw. ein Vielfaches davon betragen.

Gemäß einer bevorzugten Ausführungsform bleibt die Propellerachse bei einer Rotation des C-Arms um die C-Arm Achse unverändert.

Eine praktische Ausgestaltung einer erfindungsgemäßen Röntgeneinrichtung umfasst eine erste Halterung für die Röntgenquelle und den Röntgendetektor. Ferner ist diese erste Halterung um eine erste Achse drehbar und mit einer zweiten Halterung verbunden. Die zweite Halterung ist um eine zweite Achse drehbar und mit einer dritten Halterung verbunden. Die dritte Halterung ist entweder starr oder drehbar mit dem Gebäude verbunden oder aber dreh- oder verschiebbar mit einer Kette von einer oder mehreren weiteren Halterungen, die nacheinander durch Gelenke und schließlich mit dem Gebäude verbunden sind. Die erste und die zweite Achse schneiden sich in einem Punkt. Falls die dritte Halterung drehbar mit dem Gebäude oder einer vierten Halterung verbunden ist, so schneidet auch diese dritte Achse den Schnittpunkt der beiden ersten Achsen. Die Bewegungen um die beiden ersten Drehachsen erfolgen motorisch und gesteuert. Falls vorhanden, dienen die vierte und alle weiteren Halterungen dazu, den Schnittpunkt der beiden ersten Achsen in die Nähe des Untersuchungsobjektes zu bringen.

Diese Röntgeneinrichtung kann vorzugsweise so ausgsstaltet sein, dass die erste Achs-Halterung ein C-Arm ist, an dessen Enden Röntgenquelle bzw. -detektor befestigt sind. Die zweite Halterung trägt diesen C-Arm mit Hilfe einer kreisförmig gebogenen Schiene, so dass der C-Arm um die erste, nun als C-Arm Achse bezeichnete Achse gedreht werden kann. Die Drehung um die zweite Achse führt zu einer propellerartigen Bewegung des C-Armes um die Propellerachse. Die C-Arm Achse und die Propellerachse stehen aufeinander senkrecht und besitzen einen gemeinsamen Schnittpunkt, das so genannte Isozentrum. Auch die gerade Verbindungslinie zwischen dem Brennfleck der Röntgenröhre und dem Mittelpunkt des Detektors geht durch das Isozentrum. Die Röntgenquelle wird bewegt, indem der C-Arm um seine C-Arm Achse und die Halterung des C-Armes um die Propellerachse gedreht werden. Für eine vollständige Trajektorie sind beide Drehungen erforderlich Die Anordnung der Rotationsachsen bewirkt, dass die Trajektorie der Röntgenquelle, von kleinen mechanischen Ungenauigkeiten abgesehen, auf der Oberfläche einer im Isozentrum zentrierten Kugel bewegt wird Das Untersuchungsobjekt wird im Bereich des Isozentrums untergebracht.

Die erste Halterung kann wiederum bogenförmig ausgsführt sein, ist jedoch mit der zweiten Halterung über ein Drehgelenk verbunden. Die dritte Halterung trägt die zweite Halterung mittels einer kreisförmig gebogenen Schiene, so dass eine Drehbewegung möglich ist. Im Vergleich zur ersten Ausführung ist hierbei die Reihenfolge der Propellerachse und der C-Arm Achse vertauscht.

Weitere Ausgestaltungen des erfindungsgemäßen Verfahrens und der erfindungsgemäßen Vorrichtung ergeben sich aus den Unteransprüchen. Es sei an dieser Stelle darauf hingewiesen, dass die erfindungsgemäße Röntgeneinrichtung in gleicher oder ähnlicher Weise weitergebildet sein kann wie das erfindungsgemäße Verfahren und wie in den direkt oder indirekt auf Anspruch 1 rückbengenen Ansprüchen angegeben ist.

Bevorzugte Ausführungsbeispiele der vorliegenden Erfindung werden unter Bezugnahme auf die bagsfügten Figuren nachfolgend beschrieben. Es zeigen:

| | |
|---|---|
| Figur 1 | eine anschauliche Darstellung der Konstruktion einer Kugelkappe für einen Trajektorienpunkt, |
| Figuren 2a-2d | eine halbkreisförmige Trajektorie gemäß dem Stand der Technik mit den zugehörigen Kugelkappen der Trajektorie, |
| Figuren 3a, 3b | eine kreisförmige Trajektorie gemäß dem Stand der Technik mit den zugehörigen Kugelkappen der Trajektorie, |
| Figuren 3c, 3d | eine einen 3/4-Kreis darstellende Trajektorie gemäß dem Stand der Technik mit den zugehörigen Kugelkappen der Trajektorie, |
| Figuren 4a, 4b | eine Trajektorie aus zwei zueinander orthogonalen Halbkreisen mit den zugehörigen Kugelkappen der Trajektorie, |
| Figuren 4c, 4d | eine aus zwei orthogonalen Kreisen bestehende Trajektorie gemäß dem Stand der Technik mit den zugehörigen Kugelkappen der Trajektorie, |
| Figur 5 | eine Seitenansicht einer Röntgeneinrichtung gemäß einem ersten Ausführungsbeispiel der vorliegenden Erfindung, |
| Figur 6 | eine schematische Ansicht der Röntgeneinrichtung gemäß Figur 5, |
| Figur 7 | eine Seitenansicht einer Röntgeneinrichtung gemäß einem zweiten Ausführungsbeispiel der vorliegenden Erfindung, |
| Figur 8 | eine schematische Ansicht der Röntgeneinrichtung gemäß Figur 7, |
| Figur 9 | eine Tabelle, die die Winkelfunktionen $\theta_1$, $\theta_2$, $\theta_3$ unterschiedlicher Trajektorien für die Röntgeneinrichtung gemäß dem ersten Ausführungsbeispiel (linke Spalte) und für die Röntgeneinrichtung gemäß dem zweiten Ausführungsbeispiel (rechte Spalte) angibt, |
| Figuren10a, 10b | eine perspektivische Ansicht einer erfindungsgemäßen Trajektorie, die eine erste sphärische Spirale darstellt, und die zugehörigen Kugelkappen der Trajektorie, |
| Figuren 11a-11e | eine erfindungsgsmäße Trajektorie, die eine zweite sphärische Spirale darstellt, die zugehörigen Kugelkappen der Trajektorie und drei Seitenansichten der Trajektorie, |
| Figuren 12a, 12b | eine perspektivische Ansicht einer erfindungsgemäßen Trajektorie, die eine 3/4-sphärische Spirale darstellt, und die zugehörigen Kugelkappen der Trajektorie, |
| Figuren 13a-13e | eine erfindungsgemäße Trajektorie, die eine halbe verbogene Acht darstellt, die zugehörigen Kugelkappen der Trajektorie und drei Seitenansichten der Trajektorie, |
| Figuren 14a, 14b | eine perspektivische Ansicht einer erfindungsgemäßen Trajektorie, die einen ersten verbogenen Kreis darstellt, und die zugehörigen Kugelkappen der Trajektorie. |
| Figuren 15a, 15b | eine perspektivische Ansicht einer erfindungsgemäßen Trajektorie, die einen zweiten verbogenen Kreis darstellt, und die zugehörigen Kugelkappen der Trajektorie, |
| Figuren 16a-16e | eine erfindungsgemäße Trajektorie, die zwei miteinander verbundene sphärische Spiralen darstellt, die zugehörigen Kugelkappen der Trajektorie und drei Seitenansichten der Trajektorie, |

Figuren 17a, 17b eine perspektivische Ansicht einer erfindungsgemäßes Trajektorie, die zwei miteinander verbundene gebogene Kreise darstellt, und die zugehörigen Kugelkappen der Trajektorie,

Figuren 18a-18e eine perspektivische Ansicht einer erfindungsgemäßen Trajektorie, die eine verbogene Acht darstellt, die zugehörigen Kugelkappen der Trajektorie und drei Seitenansichten der Trajektorie, und

Figuren 19a,19b eine erfindungsgemäße Trajektorie, die eine verbogene Doppelschleife darstellt, und die zugehörigen Kugelkappen der Trajektorie.

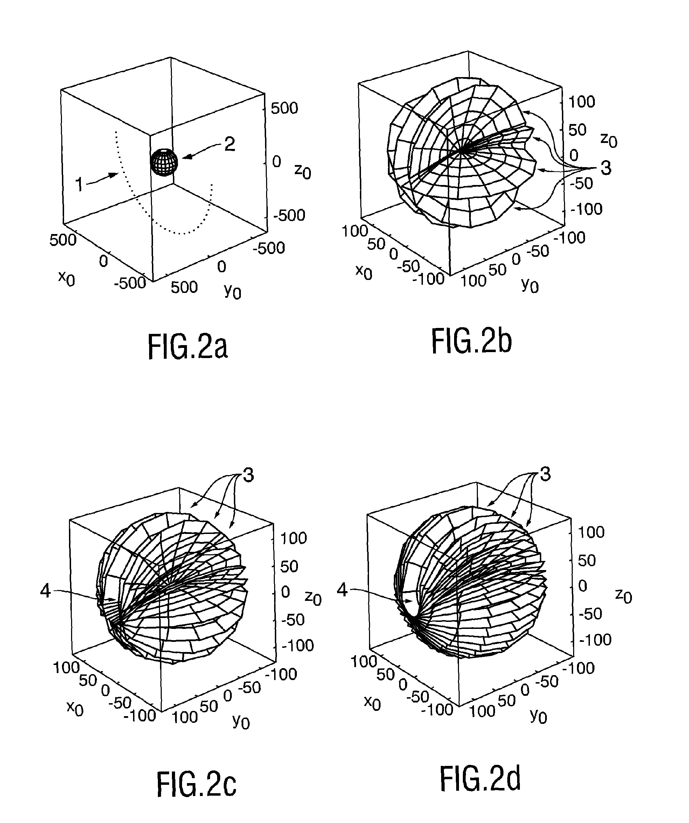

Fig. 2a zeigt eine bekannte halbkreisförmige Trajektorie 1 sowie den kugelförmigen Untersuchungsbereich 2. Fig. 2b bis Fig. 2d illustrieren die Füllung der Kugel 2 mit Kugelkappen 3. In Fig. 2b sind vier Kugelkappen 3 dargastellt, in Fig. 2c sind 12 Kugelkappen 3 dargestellt, in Fig 2d sind 19 Kugelkappen 3 dargestellt. Die zu den Kugelkappen gehörenden Trajektorienpunkte wurden dabei jeweils gleichmäßig entlang der Trajektorie verteilt. Aus Fig. 2b bis Fig. 2d ist ersichtlich, dass eine halbkreisförmige Trajektorie 1 nicht vollständig ist, denn es ist erkennbar, dass ein keilförmiger Bereich 4 der zu füllenden Kugel 2 von Kugelkappen 3 ausgäspart bleibt.

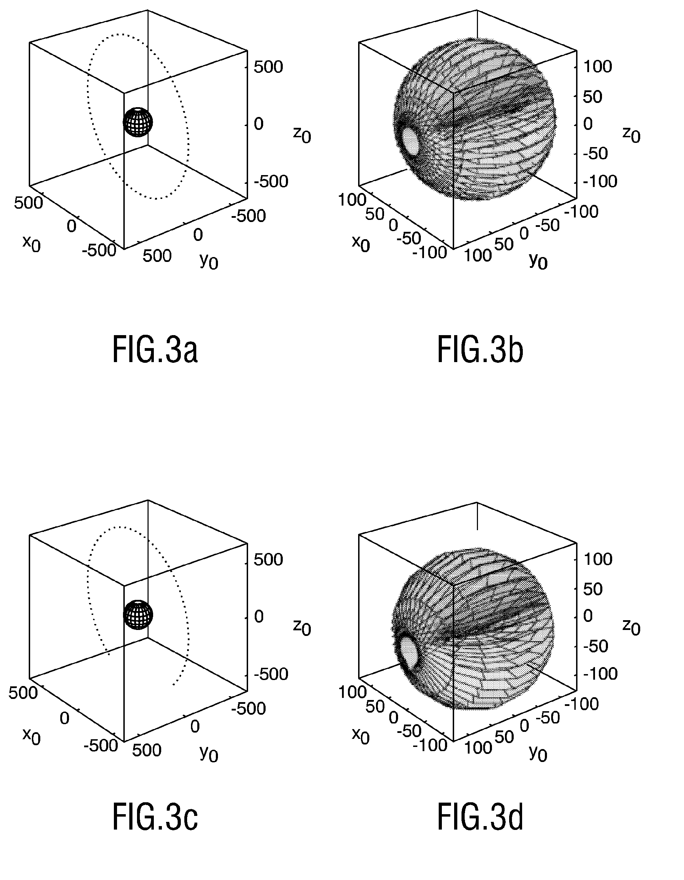

Fig. 3a und 3c zeigen zwei ebenfalls bekannte Trajektorien. In Fig. 3a ist eine kreisförmige Trajektorie dargestellt. Fig. 3b zeigt 36 Kugelkappen der in Fig. 3a dargestellten Trajektorie. Es ist zu erkennen, dass ein Bereich der zu füllenden Kugal von Kugelkappen ausgespart bleibt. Dieser Bereich bildet eine doppelt konische Region. Fig. 3c zeigt eine Trajektorie, die einem 3/4-Kreis entspricht. Die in Fig. 3d gezeigten 28 Kugelkappen für den Dreiviertelkreis können den vorstehend beschriebenen, doppelt kegelförmigen Bereich nicht abdecken. Die in Fig. 3a und 3c gezeigten Trajektorien sind daher nicht vollständig.

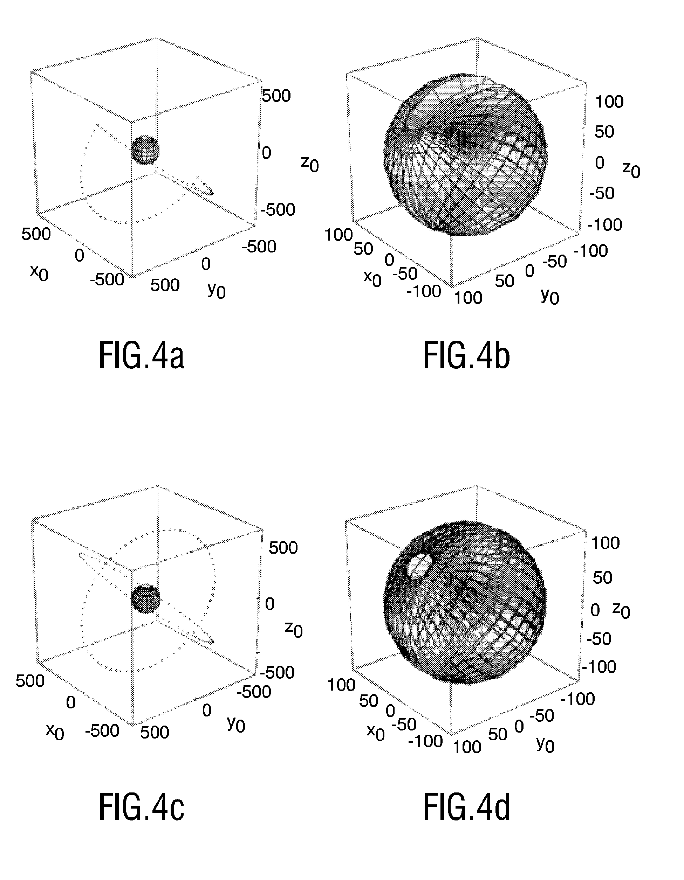

Fig. 4a und 4c zeigen weitere bekannte Trajektorien. Fig. 4a zeigt eine Trajektorie, die zwei orthogonale Halbkreise darstellt. Fig. 4b zeigt 36 Kugelkappen für die Trajektorie in Fig. 4a. Es ist wiederum ersichtlich, dass diese Trajektorie nicht vollständig ist. Fig. 4c zeigt eine Trajektorie, die zwei orthogonale Kreise darstellt. Fig. 4d zeigt 72 Kugelkappen für die Trajektorie in Fig. 4c. Im Gegensatz zu den vorangegangenen Beispielen ist diese Trajektorie vollständig.

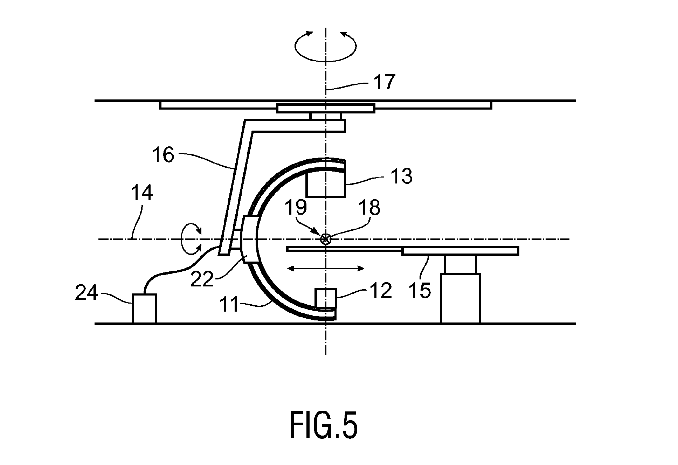

Fig. 5 zeigt eine schematische Seitenansicht der Röntgeneinrichtung gemäß einem ersten Ausführungsbeispiel der vorliegenden Erfindung. Eine Röntgenquelle 12 und ein Röntgendetektor 13 sind an einander gegenüberliegenden Seiten des C-Arms 11 befestigt. Dieser ist um eine Propellerachse 14 und eine C-Arm Achse 19 mittels einer C-Arm-Halterung 22 rotierbar gelagert. Die C-Arm Achse 19 ist in der vorliegenden Darstellung senkrecht zur Zeichenebene orientiert und geht durch ein Isozentrum 18. Eine gerade Verbindungslinie zwischen dem Brennpunkt bzw. Zentrum der Röntgenquelle 12 und dem Zentrum des Röntgendetektors 13 schneidet die Propellerachse 14 und die C-Arm Achse 19 im Isozentrum 18. Der C-Arm 11 ist mittels eines L-Arms 16 um eine L-Arm Achse 17 drehbar gelagert. Die L-Arm Achse 17 schneidet die Propellerachse 14 und die C-Arm Achse 19 im Isozentrum 18. Ort und Orientierung der L-Arm Achse 17 und der geraden Verbindungslinie zwischen Röntgendetektor 13 und Röntgenquelle 12 entsprechen bei der gezeigten Grundstellung einander. Zur Steuerung der Röntgeneinrichtung ist eine Steuereinheit 24 vorgesehen.

Im Unterschied zu der Röntgeneinrichtung INTEGRIS V5000 kann der C-Arm 11 mit dem Röntgendetektor 13 und der Röntgenquelle 12 um die C-Arm Achse 19 rotiert werden, während gleichzeitig die C-Arm Halterung 22 um die Propellerachse 14 gedreht wird und Projektionsabbildungen des Untersuchungsobjektes aufgenommen werden. Die beiden Drehbewegungen erfolgen motorisch und gesteuert. Auf diese Weise kann die Röntgenquelle 12 entlang einer vorgegebenen Bahn um das Untersuchungsobjekt im Bereich des Isozentrums 18 bewegt werden.

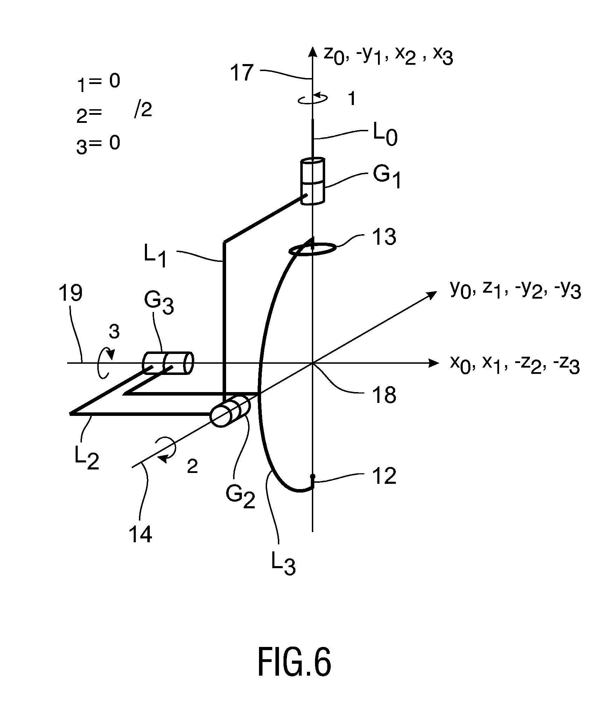

Figur 6 zeigt eine schematisierte Darstellung der Röntgeneinrichtung gemäß dem in Figur 5 gezeigten ersten Ausführungsbeispiel. Dabei sind feste Halterungen L mittels Drehverbindungen G in der folgenden Weise miteinander verbunden. Die Halterung $L_0$ entspricht der L-Arm-Aufhängung und ist beispielsweise an der Gebäudedecke befestigt. Halterung $L_1$ ist der L-Arm (16 in Figur 5). Halterung $L_2$ ist die C-Arm-Aufhängung (22 in Figur 5). Halterung $L_3$ ist der C-Arm (11 in Figur 5). Verbindungsglied $G_1$ verbindet die Halterungen $L_0$ und $L_1$. Verbindungsglied $G_2$ verbindet die Halterungen $L_1$ und $L_2$. Verbindungsglied $G_3$ verbindet die Halterungen $L_2$ und $L_3$. Jedes Verbindungsglied definiert dabei eine der Rotationsachsen, die sich im Isozentrum schneiden.

Wie in der Robotertechnik üblich, wird ein rechtshändiges kartesisches $(x_k, y_k, z_k)$-Koordinatensystem mit den Halterungen $L_k$ (für k = 0, 1, 2, 3) eingeführt. Diese vier Koordinatensysteme bewegen sich mit den Halterungen. Der Ursprung all dieser vier Koordinatensysteme fällt mit dem Isozentrum 18 zusammen, und die Achsen sind wie in Figur 6 gezeigt orientiert. Der Winkel zwischen der positiven $x_{k-1}$-Achse und der positiven $x_k$-Achse wird als $\theta_k$ (für k =1, 2, 3) bezeichnet. Jeder dieser Winkel kann innerhalb eines vorgegebenen Winkelbereiches jeden beliebigen Wert annehmen. Jedes $(\theta_1, \theta_2, \theta_3)$-Tripel beschreibt eine Konfiguration der gezeigten Röntgeneinrichtung. In Figur 6 ist die Basiskonfiguration gezeigt, die durch die Winkel $\theta_1 = 0$, $\theta_2 = -\pi/2$ und $\theta_3 = 0$ charakterisiert ist. Ein Punkt im Raum kann durch seine Koordinaten $\mathbf{x}_3 = (x_3, y_3, z_3)$ in dem mit der Halterung $L_3$ verbundenen Koordinatensystem beschrieben werden, aber auch durch seine Koordinaten $\mathbf{x}_2 = (x_2, y_2, z_2)$, durch seine Koordinaten $\mathbf{x}_1 = (x_1, y_1, z_1)$ und durch

seine Koordinaten $\mathbf{x}_0 = (x_0, y_0, z_0)$.

Die Übertragung von einem Koordinatensystem in das nächste wird durch das Matrix-Vektor-Produkt folgendermaßen beschrieben:

$$x_{k-1} = R_k(\theta_k)x_k$$

für k = 1, 2, 3
wobei $R_k(\theta_k)$ eine einfache bekannte Rotationsmatrix ist.

Die Röntgenquelle wird bewegt, indem die Winkel $\theta_1$, $\theta_2$ und $\theta_3$ verändert werden, etwa als Funktion eines reellen Parameters s, der ein Intervall $[s_-, s_+]$ durchläuft. In Lehrbüchern der Robotik wird gezeigt, dass man die Trajektorie $\mathbf{a}(s)$ der Röntgenquelle bei gegebenen Winkeln $\theta_1(s)$, $\theta_2(s)$ und $\theta_3(s)$ durch die Formel

$$a(s) = R(\theta_1(s),\theta_2(s),\theta_3(s))x_{src}, \ s \in [s_-,s_+]$$

berechnen kann. Hierbei ist $\mathbf{x}_{src}$ ein Vektor und $\mathbf{R}(\theta_1(s),\theta_2(s),\theta_3(s))$ eine Rotationsmatrix. Im einzelnen gilt $\mathbf{x}_{src} = (-d_{src}, 0, 0)$, wobei $d_{src}$ den Abstand der Röntgenquelle vom Isozentrum bezeichnet. Die Rotationsmatrix hat die Form

$$\mathbf{R}(\theta_1, \theta_2, \theta_3) =$$

$$
\begin{array}{lll}
\cos\theta_1\cos\theta_2\cos\theta_3 - \sin\theta_1\sin\theta_3 & -\cos\theta_1\cos\theta_2\sin\theta_3 - \sin\theta_1\cos\theta_3 & \cos\theta_1\sin\theta_2 \\
\sin\theta_1\cos\theta_2\cos\theta_3 + \cos\theta_1\sin\theta_3 & -\sin\theta_1\cos\theta_2\sin\theta_3 + \cos\theta_1\cos\theta_3 & \sin\theta_1\sin\theta_2 \\
-\sin\theta_2\cos\theta_3 & \sin\theta_2\sin\theta_3 & \cos\theta_2
\end{array}
$$

Da die Bewegung der Röntgenquelle 12 entlang einer Trajektorie ausschließlich durch Multiplikation mit einer Rotationsmatrix erzeugt werden kann, befindet sich die Trajektorie der Röntgenquelle 12 auf einer Kugeloberfläche um den Ursprung des Koordinatensystems, also um das Isozentrum 18.

Der Parameter s entspricht nicht notwendigerweise der Zeit. Die Trajektorie kann auch durch einen anderen Parameter beschrieben werden, sofern dieser durch eine Parametertransformation aus dem Parameter s hervorgeht. Parametertransformationen sind bijektive stetige Abbildungen. Die Parameterisierung der Kurve nach der Zeit erfolgt dadurch, dass eine geeignete Parametertransformation s(t) gefunden wird. Um einen realistischen Verlauf der Röntgenquelle 12 entlang der Trajektorie mit der Zeit t zu beschreiben, muss die Parametertransformation zudem differenzierbar sein. Denn die Geschwindigkeit der Röntgenquelle 12 auf der Trajektorie ist nichts anderes als die Ableitung von $\mathbf{a}(s)$ nach der Zeit.

Zusammenfassend sei noch einmal gesagt, dass der C-Arm 11 und die C-Arm Halterung der Röntgeneinrichtung gemäß dem ersten Ausführungsbeispiel motorisch und gesteuert gleichzeitig um die C-Arm Achse 19 bzw. die Propellerachse 14 drehbar sind. Währenddessen können Projektionsabbildungen des abzubildenden Objekts aufgenommen werden. Wichtig dabei ist, dass für jede Projektionsabbildung der Ort der Röntgenquelle 12 auf der Trajektorie bekannt ist. Vorzugsweise ist die Propellerachse 14 des ersten Ausführungsbeispiels derart ausgebildet, dass der C-Arm 11 um bis zu 270°, bis zu 360° oder um ein Vielfaches von 360° um die Propellerachse 14 rotiert werden kann.

Die vorstehenden Beschreibungen des Ortes der Röntgenquelle 12 mittels eines im Isozentrum angeordneten dreidimensionalen kartesischen Koordinatensystems ist nicht zwingend. Jedes dreidimensionale Koordinatensystem, beispielsweise ein Kugelkoordinatensystem, ist dazu geeignet den Ort der Röntgenquelle 12 zu beschreiben. Ferner kann dieselbe Trajektorie auf unterschiedliche Weise parameterisiert werden bzw. mit unterschiedlicher Geschwindigkeit von einer Röntgenquelle 12 durchfahren werden.

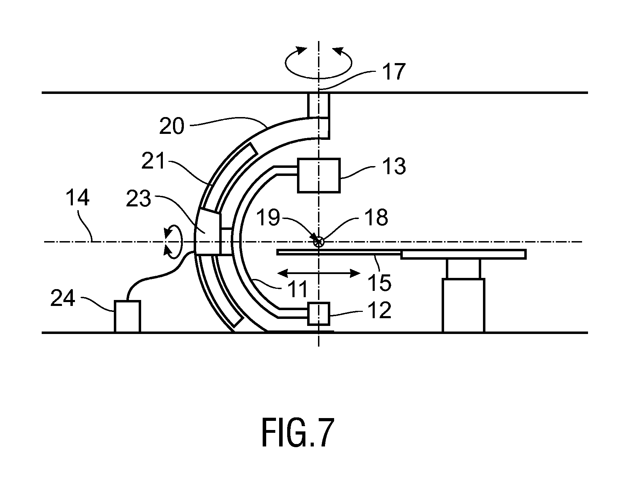

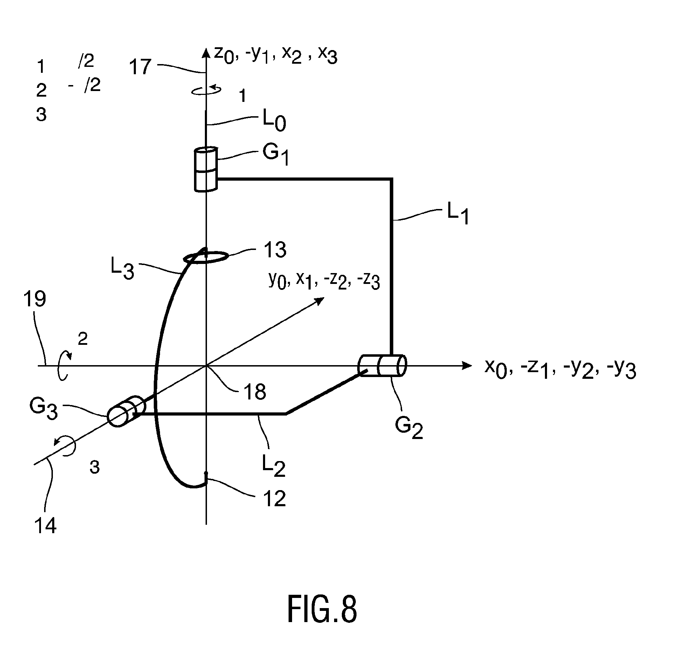

Durch den beschriebenen Formalismus sind jedoch alle möglichen Trajektorien auf einer Kugeloberfläche beschreibbar. Erfindungsgemäß werden solche Trajektorien ausgewählt, bei denen mindestens zwei Winkel während der Erfassung der Projektionsabbildungen variieren. Würde nur ein Winkel variieren, so ergäbe sich eine planare Trajektorie, die in einer einzigen Ebene läge und nicht zu einer Erfüllung der Vollständigkeitsbedingung führen würde. Der beschriebene Formalismus lässt sich direkt durch die in den Figuren 5 und 6 gezeigte Röntgeneinrichtung sowie auch durch die nachfolgend beschriebene, in den Figuren 7 und 8 gezeigte Röntgeneinrichtung realisieren, was besonders vorteilhaft ist.

Fig. 7 zeigt ein zweites Ausführungsbeispiel für eine Röntgeneinrichtung gemäß der vorliegenden Erfindung.

Die in Fig. 7 verwendeten Bezugszeichen kennzeichnen dieselben Merkmale wie in Fig. 5. Die C-Arm Achse 19 liegt ebenfalls senkrecht zur Blattebene und geht durch das Isozentrum 18 in der dargestellten Figur. Der C-Arm 11 ist mittels der C-Arm-Halterung 23 gelagert und kann um die Propellerachse 14 rotiert werden, während gleichzeitig die C-Arm Halterung 23 um die C-Arm Achse 19 gedreht wird und Projektionsabbildungen aufgenommen werden. Diese Möglichkeit besteht bereits bei der Röntgeneinrichtung gemäß dem ersten Ausführungsbeispiel in Fig. 5. Der entscheidende Unterschied zum ersten Ausführungsbeispiel besteht jedoch darin, dass die Propellerachse 14 bei einer Rotation um die C-Arm Achse 19 mit rotiert wird. Die Propellerachse ist daher nicht immer waagerecht. Außerdem kann der C-Arm 11 leichter ausgeführt werden, was eine schnellere Propellerbewegung erlaubt.

Fig 8 zeigt eine schematische Darstellung der Röntgeneinrichtung gemäß dem zweiten Ausführungsbeispiel. Die mit den Halterungen verbundenen Koordinatensysteme sind so ausgewählt, dass dieselbe Rotationsmatrix wie beim ersten Ausführungsbeispiel verwendet werden kann, um eine beliebige durch die Röntgeneinrichtung verfahrbare Trajektorie darzustellen. Die in Fig. 8 dargestellte Position des Dreharms ist durch die Winkel $\theta_1 = p/2$, $\theta_2 = -p/2$, $\theta_3 = 0$ charakterisiert. Der Propellerwinkel der Röntgeneinrichtung des zweiten Ausführungsbeispiels ist vorzugsweise um 360° oder um ein Vielfaches von 360° veränderbar.

Bei der Realisierung der Trajektorien ist zu beachten, dass die Bewegung entlang einer Trajektorie mit einer Beschleunigungsphase beginnen und mit einer Bremsphase enden muss. Dies kann durch eine geeignete Parametertransformation $s = s(t)$ erreicht werden, ohne die Form der Trajektorie zu ändern. Dabei wird allerdings die für das Durchlaufen der Trajektorie erforderliche Zeit vergrößert. Alternativ kann die gewünschte Trajektorie um Beschleunigungs- und Bremsphasen verlängert werden. Das interessierende Stück der verlängerten Trajektorie kann dann mit gleichmäßigerer Geschwindigkeit durchfahren werden.

Die beiden veränderbaren Winkel der Trajektorie sollten zweimal differenzierbar sein, damit die Trajektorie physikalisch realisierbar ist. Bevorzugt sollten die Winkel sogar zweimal stetig differenzierbar sein, wodurch unstetige Beschleunigungen vermieden werden. Die ersten

Ableitungen der Winkel können dabei zur Charakterisierung der Trajektorien dienen. Möglichst wenig Vorzeichenwechsel bei den Ableitungen sind erwünscht. Noch besser sind näherungsweise konstante erste Ableitungen.

Die Einstellbereiche der Drehwinkel um die C-Arm Achse und die L-Arm-Achse sind aus mechanischen Gründen häufig auf 180° oder weniger beschränkt. Es ist technisch einfacher, auch den Drehwinkel für die Propellerbewegung auf weniger als 360° zu beschränken. Allerdings ist es für geschlossene Trajektorien wünschenswert, wenn die Propellerbewegung unbeschränkt erfolgen kann. Dies wird in der Regel einen Schleifring zur Übertragung von elektrischen Signalen erfordern.

Bei der Gestaltung der Trajektorien ist zu beachten, dass die Röntgenquelle, der Röntgendetektor und andere Teile der Untersuchungseinrichtung nicht gegen das Untersuchungsobjekt und seine Halterung - in der Regel ein Patient auf dem Untersuchungstisch - stoßen.

Die Unterschiede hinsichtlich der Ausgestaltung der in den Figuren 5 und 7 gezeigten Ausführungsformen der erfindungsgemäßen Röntgeneinrichtung haben Folgen für die Trajektorien, die bei fester Stellung des L-Arms gefahren werden können. Die gezeigten Ausführungsformen können auch weiter dadurch variiert werden, dass der L-Arm nicht drehbar gestaltet ist, am Boden befestigt ist statt an der Decke und an einem verschiebbaren Schlitten angeordnet ist. Für die Drehbewegung um die Propellerachse können Schleifringe vorgesehen sein. Bei der in Figur 5 gezeigten Ausführungsform kann der C-Arm auch mehr als 180° umfassen und um mehr als 180° um die C-Arm Achse gedreht werden.

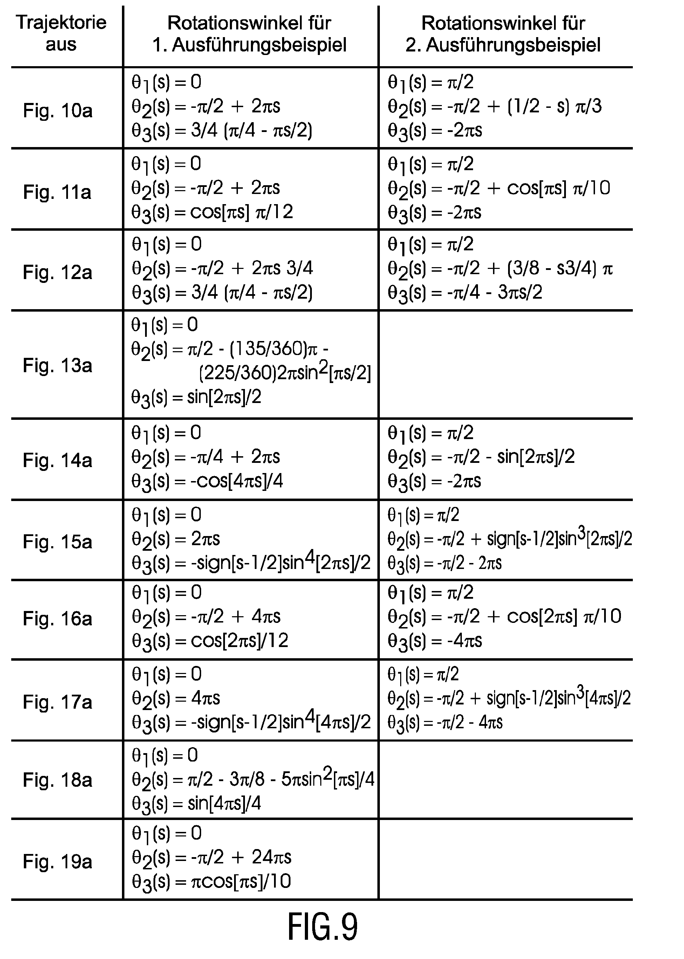

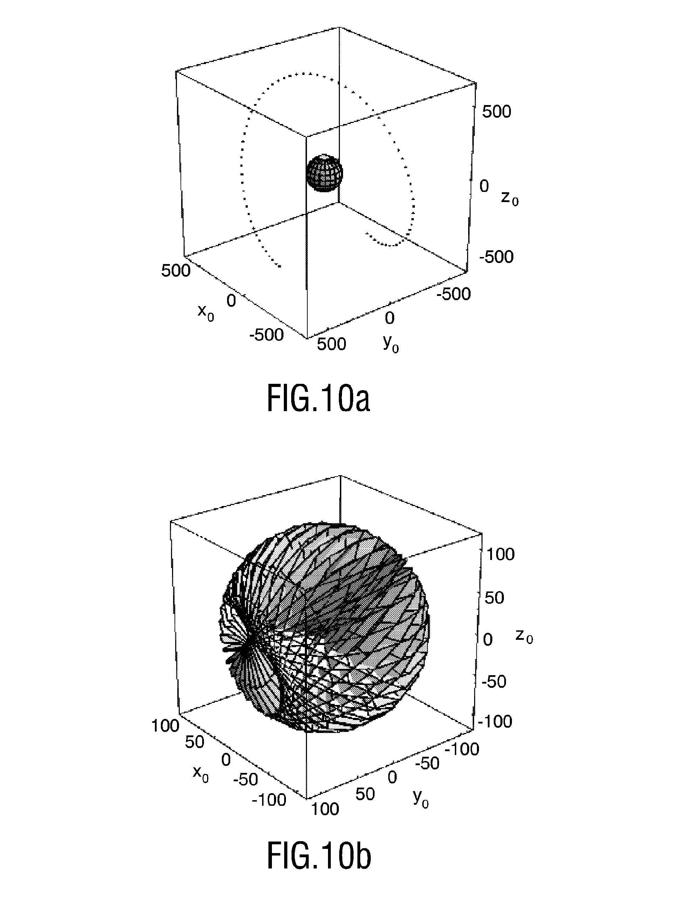

Fig 9 zeigt in tabellarischer Form die Drehwinkel $\theta_1(s)$, $\theta_2(s)$ und $\theta_3(s)$ für eine Röntgeneinrichtung gemäß dem ersten Ausführungsbeispiel und eine Röntgeneinrichtung gemäß dem zweiten Ausführungsbeispiel. Das Symbol sign steht für die Signum-Funktion, welche definiert ist durch sign[x] = +1 für $x \geq 0$ und sign[x] = -1 für x < 0. Jede der in Fig 10a bis 19a dargestellten Trajektorien wird durch die entsprechenden Rotationswinkel $\theta_1(s)$, $\theta_2(s)$ und $\theta_3(s)$ beschrieben, wenn der Parameter s das Intervall [0,1] durchläuft.

Für eine Röntgeneinrichtung gemäß dem ersten Ausführungsbeispiel gehört der Winkel $\theta_2(s)$ zur Propellerachse und der Winkel $\theta_3(s)$ zur C-Arm Achse. Bei einer Röntgeneinrichtung gemäß dem zweiten Ausführungsbeispiel gehört der Winkel $\theta_2(s)$ zur C-Arm Achse und der Winkel $\theta_3(s)$ zur Propellerachse. Der Winkel $\theta_1$ gibt die Stellung des L-Armes an und bleibt während der Erfassung der Kegelstrahlprojektionen konstant.

Im folgenden werden zumeist die für das erste Ausführungsbeispiel der Röntgeneinrichtung geltenden Varianten der Trajektorien gezeigt. Die für das zweite Ausführungsbeispiel der Röntgeneinrichtung gültigen Trajektorien sind zumeist identisch, wobei jedoch manche Trajektorien nur für das erste Ausführungsbeispiel gelten.

Die Beispieltrajektorien hängen auch von gewissen additiven und multiplikativen Konstanten ab. Diese Konstanten sind gegebenenfalls so zu wählen, dass die Vollständigkeitsbedingung erfüllt ist. Bei den Beispieltrajektorien ist der Abstand zwischen Röntgenquelle und Isozentrum 660 mm und der Radius des Untersuchungsbereichs etwa 120 mm. Ferner sind die Konstanten so gewählt, dass die Vollständigkeitsbedingung für diesen Untersuchungsbereich jeweils erfüllt ist.

Die Trajektorien in den Fig. 10a bis 19d sind in einem kartesischen Koordinatensystem dargestellt, dessen

Achsen mit $x_o$, $y_o$, $z_o$ bezeichnet sind und die den Achsen $x_o$, $y_o$, $z_o$ in den Figuren 6 und 8 entsprechen. Die Trajektorien liegen jeweils auf einer Kugeloberfläche, deren Mittelpunkt im Ursprung des Koordinatensystems liegt. Die dargestellten Trajektorien schließen jeweils einen im Ursprung des Koordinatensystems liegenden, darzustellenden Untersuchungsbereich ein. Ferner werden die in den Fig. 10 bis 19d dargestellten Trajektorien mit Hilfe der in Fig. 9 aufgeführten Winkel und der Formel $\mathbf{a}$ (s) = $\mathbf{R}$ ($\theta_1$ (s), $\theta_2$ (s), $\theta_3$ (s)) $\mathbf{x}_{src}$ für eine bestimmte und gleichmäßig über das Intervall {0,1} verteilte Anzahl von Werten des Parameters s berechnet. Die Trajektorien sind dreidimensionale Kurven, da sich die Rotationswinkel $\theta_2$ und $\theta_3$ zugleich ändern, wenn der Parameter s verändert wird. Die in den Fig. 10a bis 19d dargestellten Trajektorien erfüllen allesamt die Vollständigkeitsbedingungen für einen kugelförmigen Untersuchungsbereich von etwa 120 mm Radius.

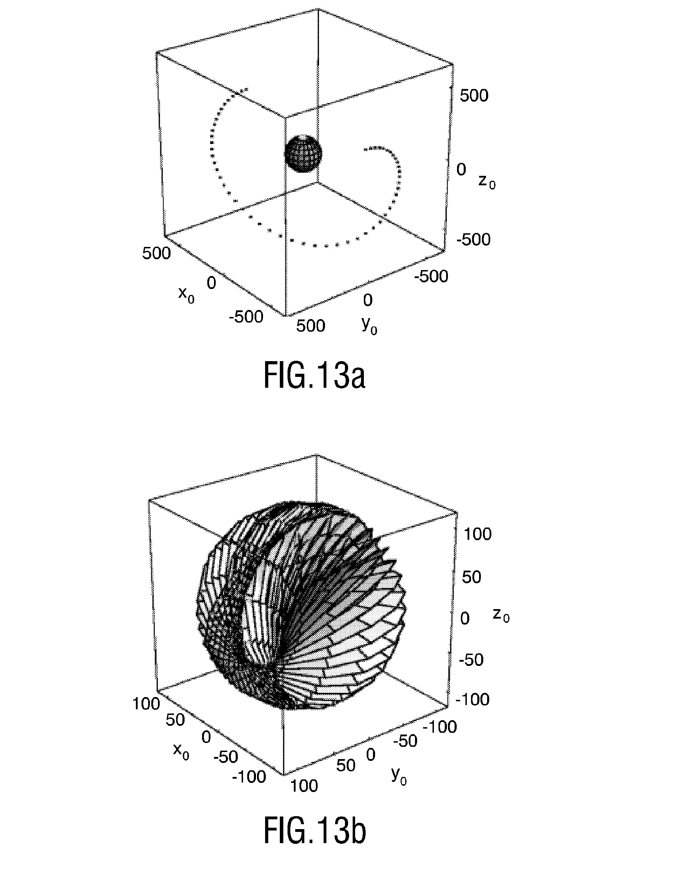

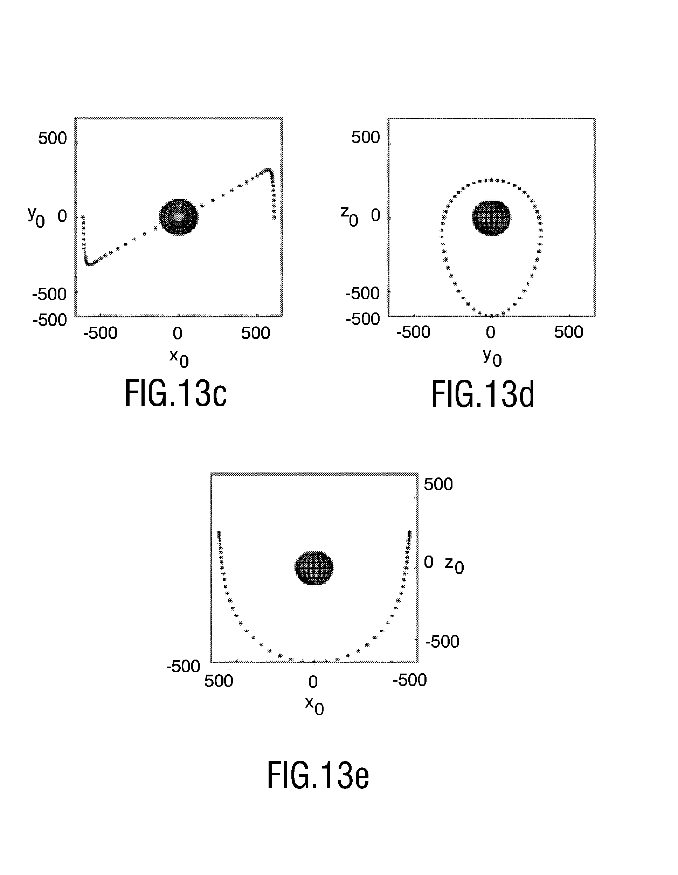

Die Fig. 10a, 11a und 12a zeigen Trajektorien, die unterschiedliche Ausführungsformen einer sphärischen Spirale darstellen. Diese gleichen im Wesentlichen einer wendelartigen Kurve, die auf eine Kugeloberfläche projiziert wird. Figur 13a zeigt eine Trajektorie, die wie eine auf eine Kugeloberfläche gelegten halbe Acht aussieht. Charakteristisch für alle diese Trajektorien ist es, dass die Ableitungen beider Drehwinkel keinen Vorzeichenwechsel aufweisen. Die Figuren 10b, 11b, 12b und 13b zeigen die zu den jeweiligen Trajektorien gehörenden Füllungen des Untersuchungsbereiches durch Kugelkappen. Es ist ersichtlich, dass jede der in Fig. 10a, 11a, 12a und 13a dargestellten Trajektorien die Vollständigkeitsbedingung erfüllt, da die entsprechenden Kugelkappen mit zunehmender Anzahl den Untersuchungsbereich lückenlos auffüllen würden.

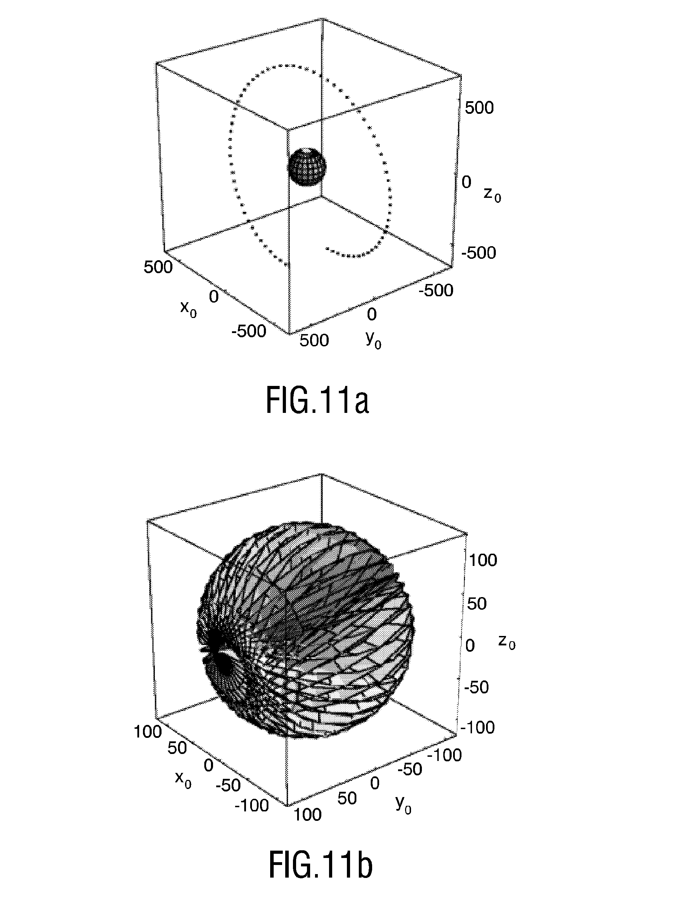

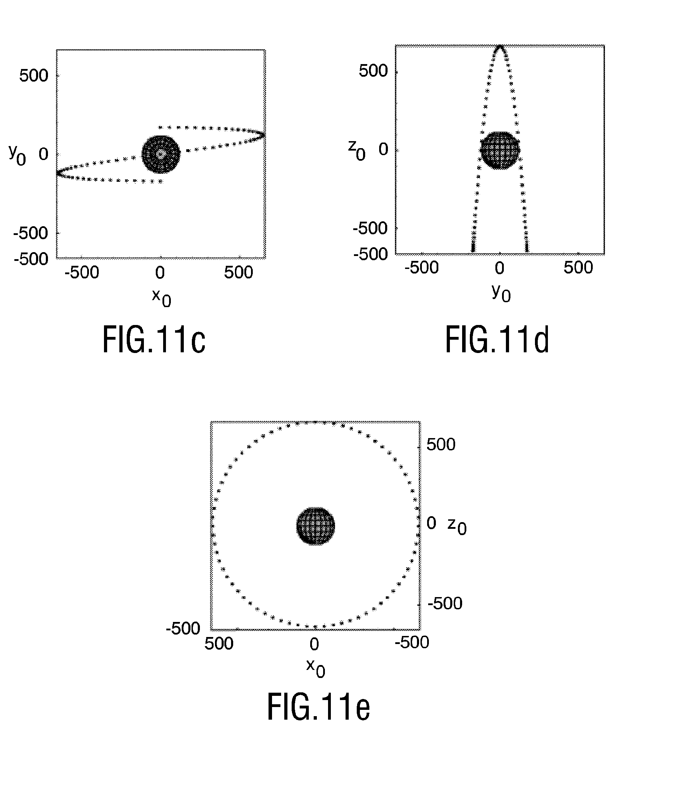

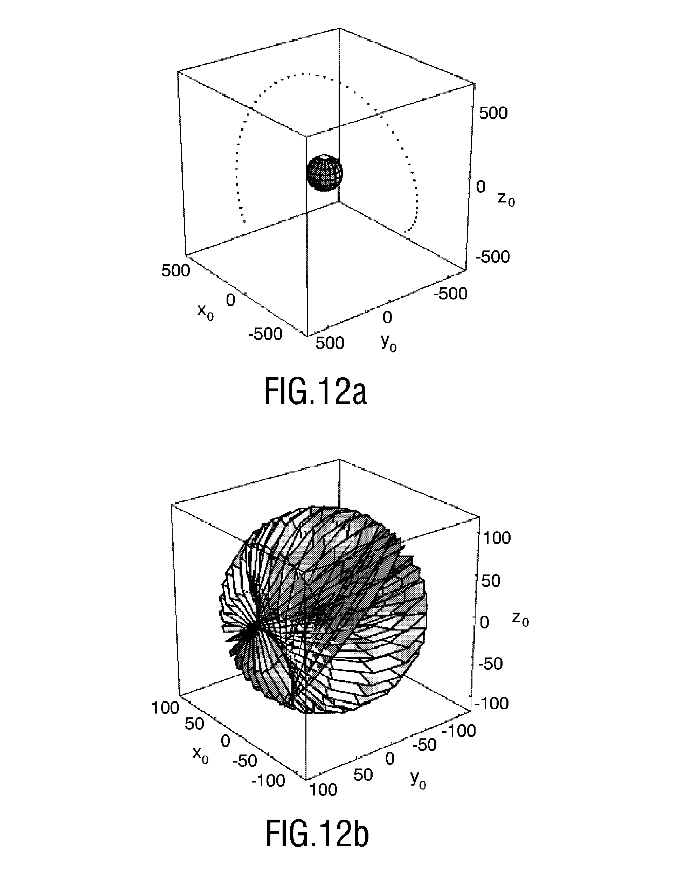

Fig. 11c, Fig. 11d und Fig. 11e zeigen den Verlauf der in Fig. 11a dargestellten Trajektorie aus einer anderen Blickrichtung. Aus der $y_0$-Richtung betrachtet, erscheint die Trajektorie in Fig. 11e als ein geschlossener Kreis. Die Röntgenquelle, die entlang dieser Trajektorie verfahren wird, vollzieht demnach eine Rotation 360° um die z-Achse. Fig. 11c zeigt die Erscheinung der Trajektorie betrachtet aus der $z_0$-Richtung. Wird die Röntgenquelle in Richtung der y-Achse verfahren, so wird sie gleichzeitig in Richtung der x-Achse verfahren, und zwar zunächst zu negativen x-Werten und anschließend zu positiven x-Werten. Der Verlauf der in Fig. 11a dargestellten Trajektorie aus $x_0$-Richtung betrachtet ist in Fig. 11d dargestellt. Die in Fig. 12a dargestellte, helixartige Trajektorie unterscheidet sich von den in Fig. 10a und Fig. 11a dargestellten helixartigen Trajektorien dadurch, dass die entlang der Trajektorie verfahrene Röntgenquelle keine vollständige Rotation um die z-Achse erfährt. Die in Fig. 12a dargestellte Trajektorie erscheint aus der x-z-Ebene betrachtet nicht als vollständiger Kreis, sondern als 3/4-Kreis.

Die Figuren 13c, 13d und 13e zeigen den Verlauf der in Figur 13a dargestellten Trajektorie wiederum aus den drei Blickrichtungen entlang der $z_0$-, $x_0$- und $y_0$-Achsen, um den Verlauf der Trajektorie einfacher vorstellbar zu machen.

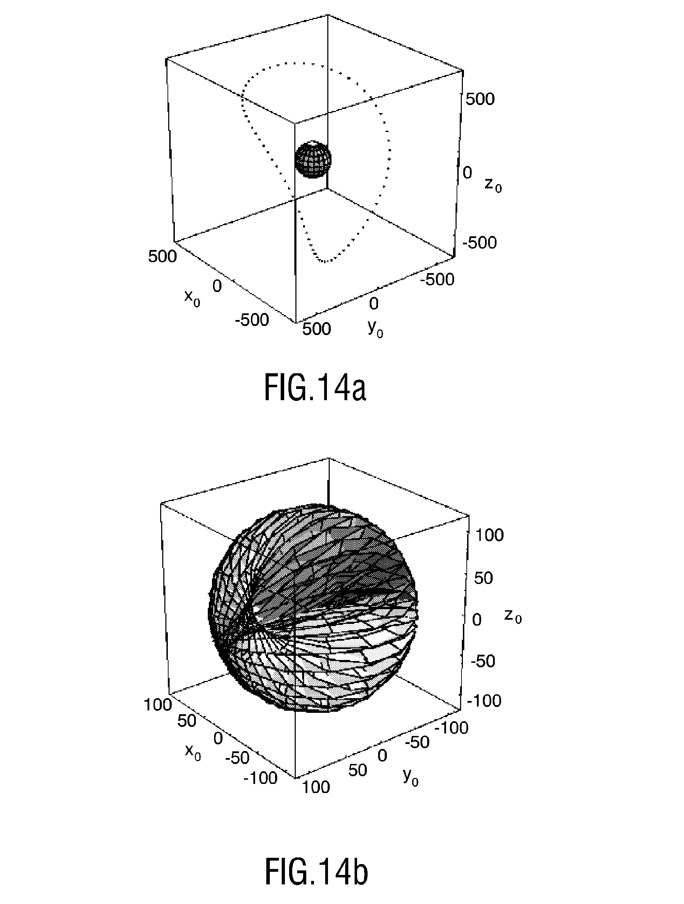

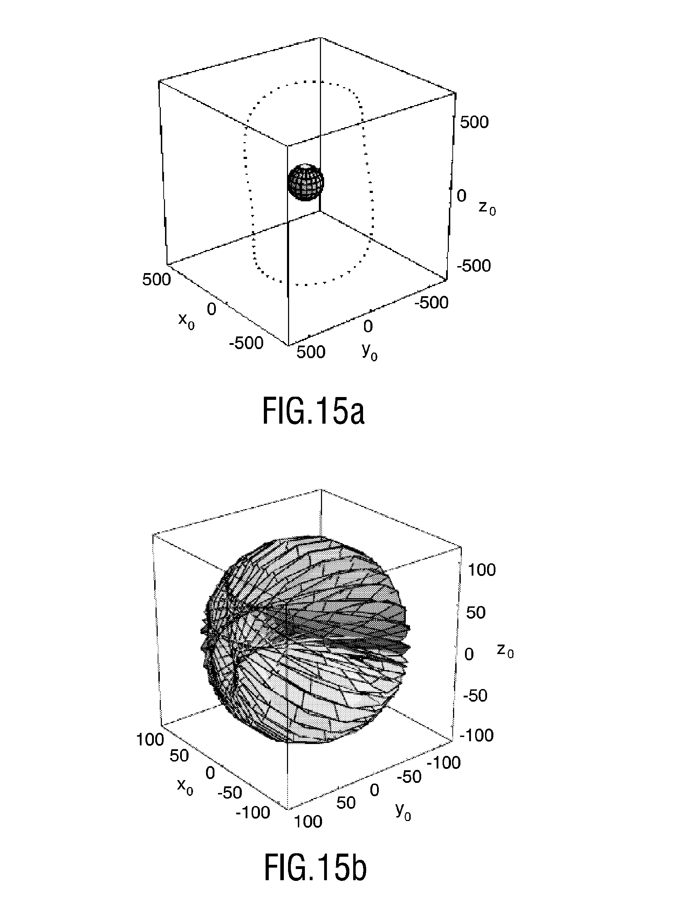

Fig. 14a und Fig. 15a zeigen erfindungsgemäße Trajektorien, die jeweils einen auf einer Kugeloberfläche liegenden, verbogenen Kreis darstellen. Charakteristisch für die Trajektorien ist eine Drehung um 360° ohne Vorzeichenwechsel der ersten Ableitung um eine der beiden Achsen und eine periodische Bewegung mit Vorzeichenwechsel um die andere Achse. Figuren 14b und 15b zeigen die den vorstehenden Trajektorien entsprechende Füllung des Untersuchungsbereiches mit Kugelkappen. Beide Trajektorien erfüllen offenbar die Vollständigkeitsbedingung. Sie stellen zudem geschlossene Kurven dar, so dass eine Röntgenquelle mehrfach hintereinander entlang dieser Trajektorien kontinuierlich und ohne anzuhalten durchfahren werden kann. Das Zustandekommen dieser Trajektorien lässt sich anhand der in Fig. 9 angegebenen Formeln für die Rotationswinkel erklären. Danach wird ein Rotationswinkel jeweils um 2p verfahren, da einer der Rotationswinkel immer eine Funktion von 2ps ist, wobei s den Wertebereich von 0 bis 1 abdeckt. Ferner ist ein anderer Rotationswinkel eine periodische Funktion, und zwar derart, dass der Wert dieses Rotationswinkels nach Durchlaufen des Wertebereichs von s wieder denselben Wert einnimmt.

Bei den vorliegenden Trajektorien wurden trigonometrischen Funktionen zur Beschreibung des zweiten Rotationswinkels verwendet.

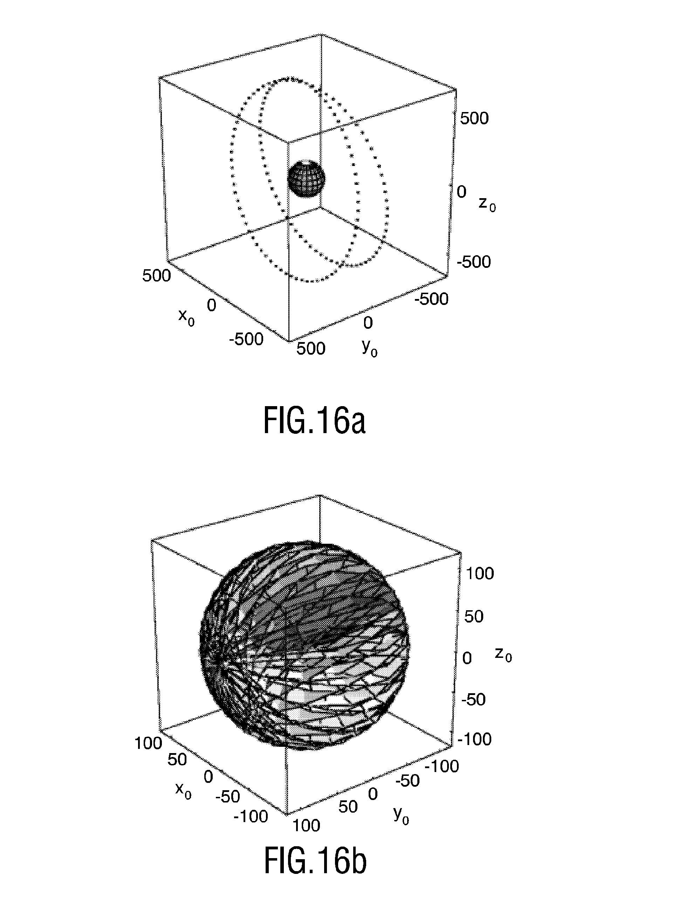

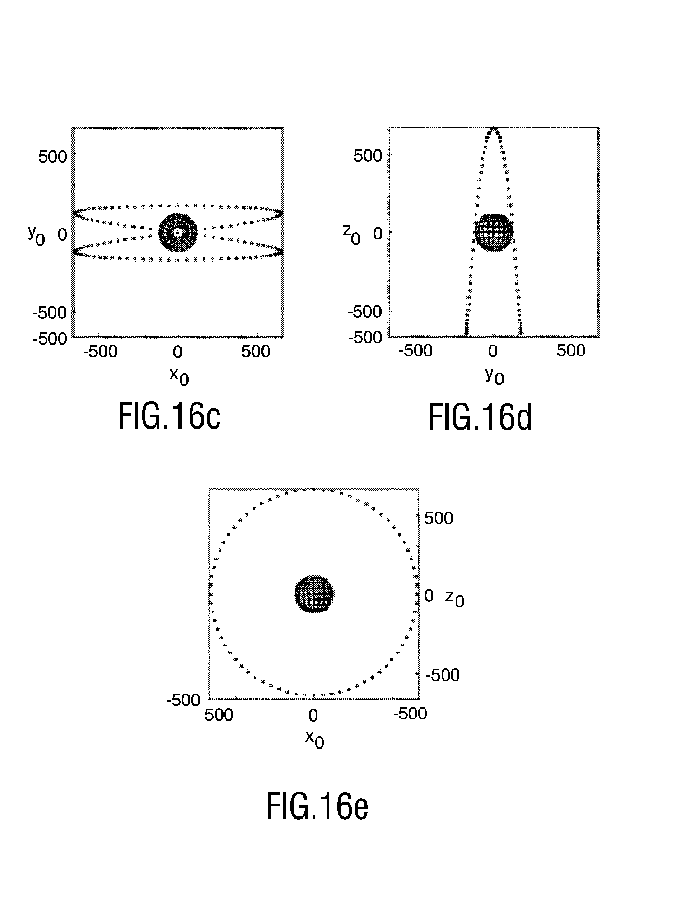

Fig. 16a zeigt eine Trajektorie, die durch das Verbinden von zwei sphärischen Spiralen der in Fig. 11a gezeigten Art zustande kommt. Aus Fig. 16b ist ersichtlich, dass auch diese Trajektorie vollständig ist. Der Vorteil dieser Trajektorie liegt darin, dass sie geschlossen ist und mit geringen Beschleunigungskräften realisiert werden kann. Fig. 16c, 16d und 16e zeigen wiederum die Trajektorie in Fig. 16a aus unterschiedlichen Blickrichtungen. Insbesondere der Vergleich der Figuren 16c und 11c ist hilfreich. Spiegelt man nämlich die in Fig. 11c dargestellte Kurve an der $y_0$-Achse, so dass alle zuvor rechts von der $y_0$-Achse liegenden Punkte nun links von der $y_0$-Achse liegen und vice versa, so erhält man eine weitere sphärische Spirale. Fügt man diese beiden Trajektorien aneinander, so erhält man die in Fig. 16c dargestellte Trajektorie.

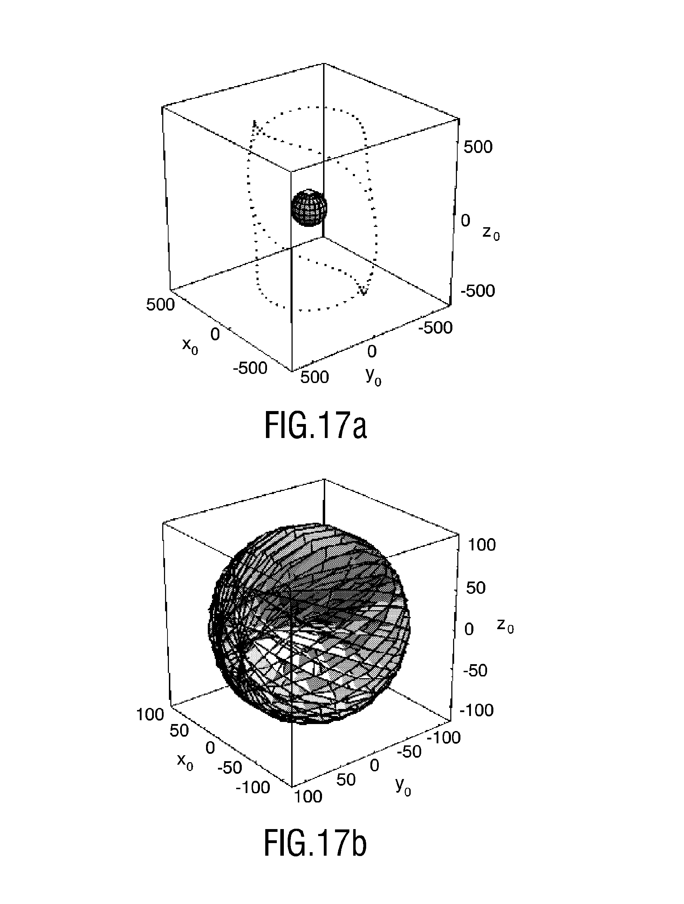

Fig. 17a zeigt eine Trajektorie, die durch Verbindung zweier verbogener Kreise zustande kommt. Figur 17b zeigt wiederum die Füllung des Untersuchungsbereiches mit Kugelkappen. Es ist zu erkennen, dass auch diese Trajektorie die Vollständigkeitsbedingung erfüllt. Vorteil dieser Verbindung ist eine symmetrische Trajektorie, was für einige Rekonstruktionsalgorithmen vorteilhaft sein kann. Die Beschleunigungskräfte sind dabei allerdings etwas größer als bei der in Figur 16a gezeigten Trajektorie.

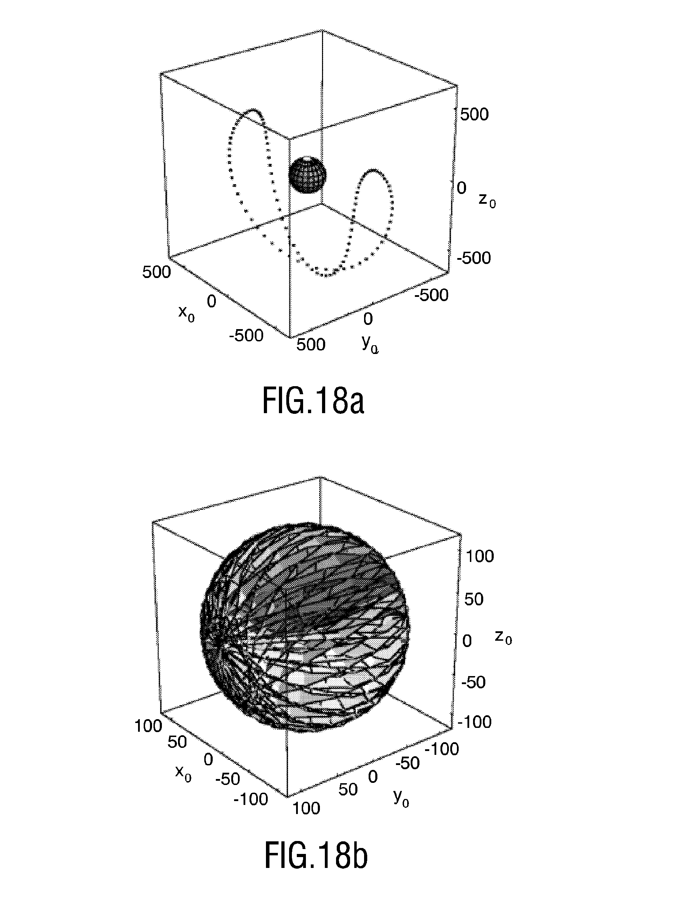

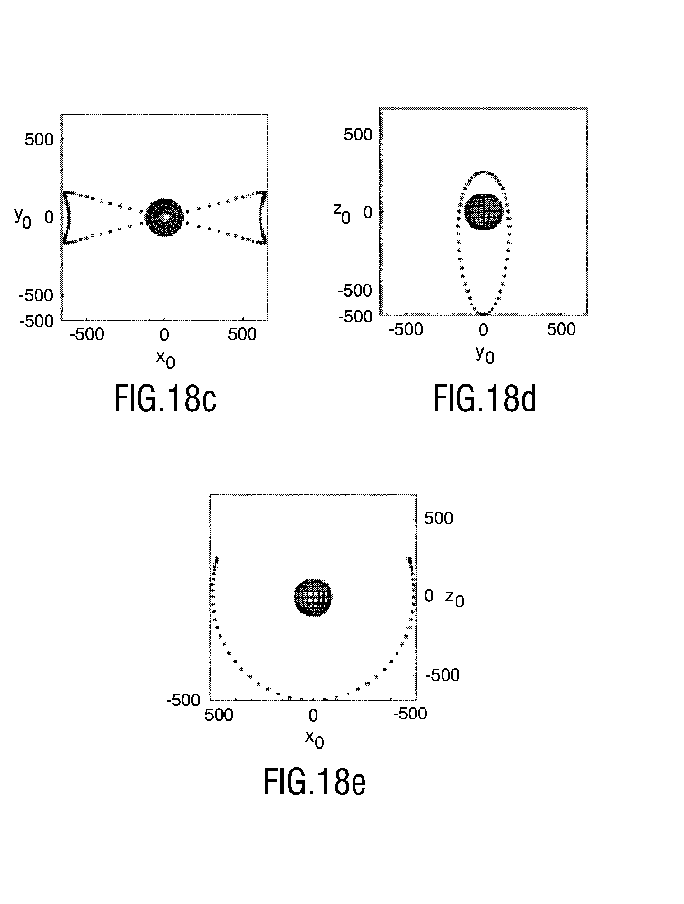

Fig. 18a zeigt eine Trajektorie, die einer auf eine Kugel gelegten Acht entspricht. Die Trajektorie ist also eine geschlossene Kurve mit zwei Schleifen, die einander gegenüber liegen, und kann als Verbindung zweier halber Achten gemäß Fig. 13a aufgefasst werden. Die exakte Beschreibung dieser Kurve ist durch die in Fig. 9 angegebenen Funktionen angegeben. Durch Fig. 18b wird wiederum bestätigt, dass die Trajektorie in Fig. 18a die Vollständigkeitsbedingung erfüllt. Fig. 18c, 18d und 18e zeigen wiederum den Verlauf der Trajektorie gemäß Fig. 18a aus drei verschiedenen Blickrichtungen. Die in Fig. 18c dargestellte Erscheinung der Trajektorie aus der $z_0$-Richtung rechtfertigt die Vorstellung einer auf eine Kugelfläche gelegten Acht. Eine linke Schleife und eine rechte Schleife sind zu sehen. Der gemeinsame Punkt der einander gegenüberliegenden Schleifen ist der unterste Punkt in Fig. 18d und Fig 18e. Die einander gegenüberliegenden Schleifen der Acht werden durch die rechts und links vom vorstehenden Punkt dargestellten Punkte wiedergegeben. Eine Röntgenquelle, die die Trajektorie durchläuft, würde aus dieser Blickrichtung beispielsweise von dem obersten rechten Punkt, durch den Ursprung, zum obersten linken Punkt und wieder zurück verlaufen. Eine solche Trajektorie kann ohne Schleif- ring mit der Röntgeneinrichtung gemäß Fig. 5 realisiert werden.

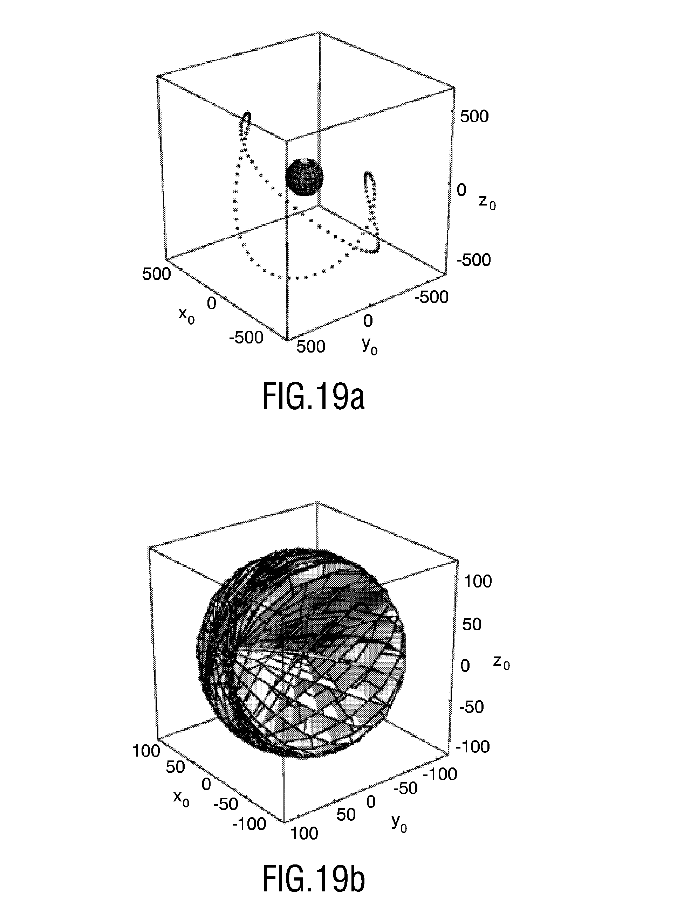

Figur 19a zeigt ein weiteres Ausführungsbeispiel einer erfindungsgemäßen Trajektorie. Aus Figur 19b ergibt sich, dass auch mit dieser Trajektorie die Vollständigkeitsbedingung erfüllt wird. Allerdings ist diese Trajektorie nur mit dem in Figur 5 gezeigten Ausführungsbeispiel der Röntgeneinrichtung realisierbar. Der L-Arm steht dabei seitlich ($\theta_1$(s) = -$\pi$/2). Mit dieser Trajektorie ist es deshalb möglich, auch den Unterkörper und die Beine eines Patienten abzubilden. Bei allen anderen Trajektorien steht der L-Arm dagegen am Kopf oder in der Nähe des Kopfes eines Patienten, weshalb mit diesen Trajektorien nur Kopf und Oberkörper abgebildet werden können. Allerdings muss bei der Doppelschleife der Winkel $\theta_2$ (Drehung um die C-Arm Achse) einen Bereich von ca. 225° überstreichen, was durch eine Verlängerung des oder der ihn haltenden Schiene erreicht werden kann.

Durch die Erfindung werden Trajektorien zur Verfügung gestellt, um einen möglichst vollständigen Satz von Projektionsabbildungen, insbesondere von Kegelstrahlprojektionen, eines in einem Untersuchungsbereich angeordneten Untersuchungsobjekts zu erfassen. Die gezeigten Trajektorien sind Beispiele hierfür. Es sind beliebig viele weitere Beispiele denkbar, mit denen die Vollständigkeitsbedingung erfüllt werden kann. Auch die gezeigten Röntgeneinrichtungen sind lediglich Beispiele, mit denen die erfindungsgemäßen Trajektorien realisiert werden können. Die Röntgeneinrichtungen können jedoch grundsätzlich auch anders ausgestaltet sein und müssen nicht zwingend einen C-Arm aufweisen.

**Patentansprüche**

1. Verfahren zur Ermittlung eines Satzes von Projektionsabbildungen für die Rekonstruktion eines dreidimensionalen Bilddatensatzes eines in einem Untersuchungsbereich (2) angeordneten Untersuchungsobjektes mittels einer eine Röntgenquelle (12) und einen Röntgendetektor (13) aufweisenden Röntgeneinrichtung, wobei die Röntgenquelle (12) zur Erfassung der Projektionsabbildungen entlang einer im Wesentlichen auf einer Kugeloberfläche liegenden Trajektorie (1) um den Untersuchungsbereich (2) geführt wird,
   **dadurch gekennzeichnet,**
   **dass** die Trajektorie (1) derart ausgestaltet ist, dass die Röntgenquelle (12) zur Ermittlung des Satzes von Projektionsabbildungen die Trajektorie kontinuierlich durchlaufen kann und dass nicht alle Punkte der Trajektorie in einer gemeinsamen Ebene liegen.

2. Verfahren nach Anspruch 1,
   **dadurch gekennzeichnet,**
   **dass** die Trajektorie (1) derart ausgestaltet ist, dass jede den Untersuchungbereich schneidende Ebene mindestens einen Punkt der Trajektorie (1) aufweist.

3. Verfahren nach Anspruch 1,
   **dadurch gekennzeichnet,**
   **dass** die Trajektorie (1) eine geschlossene Kurve darstellt.

4. Verfahren nach Anspruch 1,
   **dadurch gekennzeichnet,**
   **dass** die Trajektorie (1) eine zweimal differenzierbare Kurve darstellt.

5. Verfahren nach Anspruch 1,
   **dadurch gekennzeichnet,**
   **dass** sich die Trajektorie in der Form

$$a(s)=R(\theta_1(s),\theta_2(s),\theta_3(s))x_{src}$$

schreiben lässt, wobei $\theta_1(s)$, $\theta_2(s)$ und $\theta_3(s)$ reelle, zweimal differenzierbare Funktionen eines reellen Parameters s sind, $\mathbf{x}_{src}$ durch den Abstand $d_{src}$ der Röntgenquelle zum Mittelpunkt der Kugeloberfläche gemäß $\mathbf{x}_{src}$ = (-$d_{src}$, 0, 0) gegeben ist, $\mathbf{R}(\theta_1(s),\theta_2(s),\theta_3(s))$ die Rotationsmatrix

$$\mathbf{R}(\theta_\theta, \theta_2, \theta_3) =$$

| $\cos\theta_1\cos\theta_2\cos\theta_3 - \sin\theta_1\sin\theta_3$ | $-\cos\theta_1\cos\theta_2\sin\theta_3 - \sin\theta_1\cos\theta_3$ | $\cos\theta_1\sin\theta_2$ |
|---|---|---|
| $\sin\theta_1\cos\theta_2\cos\theta_3 + \cos\theta_1\sin\theta_3$ | $-\sin\theta_1\cos\theta_2\sin\theta_3 + \cos\theta_1\cos\theta_3$ | $\sin\theta_1\sin\theta_2$ |
| $-\sin\theta_2 \cos\theta_3$ | $\sin\theta_2 \sin\theta_3$ | $\cos\theta_2$ |

ist und $\mathbf{a}(s)$ den Ortsvektor der Trajektorie in Bezug auf ein rechtshändiges kartesisches Koordinatensystem bedeutet, dessen Ursprung mit dem Mittelpunkt der Kugeloberfläche übereinstimmt, dass mindestens zwei der Funktionen $\theta_1(s)$, $\theta_2(s)$ und $\theta_3(s)$ nicht konstant sind, und dass die Trajektorie $\mathbf{a}(s)$ derart ausgestaltet ist, dass jede Ebene, die den Untersuchungsbereich schneidet, auch die Trajektorie schneidet.

6. Verfahren nach Anspruch 5,
   **dadurch gekennzeichnet,**
   **dass** die Funktion $\theta_3(s)$ konstant ist.

7. Verfahren nach Anspruch 5,
   **dadurch gekennzeichnet,**
   **dass** die ersten Ableitungen der Funktionen $\theta_1$ (s) und $\theta_2$ (s) keinen Vorzeichenwechsel aufweisen.

8. Verfahren nach Anspruch 7,
   **dadurch gekennzeichnet,**
   **dass** die Funktionen $\theta_1$ (s) und $\theta_2$ (s) so gewählt sind, dass eine ihrer ersten Ableitungen keinen Vorzeichenwechsel und die andere mindestens einen Vorzeichenwechsel aufweist.

9. Verfahren nach Anspruch 7,
   **dadurch gekennzeichnet,**
   **dass** die Funktionen $\theta_1$ (s) und $\theta_2$ (s) so gewählt sind, dass ihre ersten Ableitungen jeweils mindestens einen Vorzeichenwechsel aufweisen.

10. Verfahren nach Anspruch 1,
    **dadurch gekennzeichnet,**
    **dass** die Projektionsabbildungen Kegelstrahlprojektionen sind.

11. Röntgeneinrichtung zur Ermittlung eines Satzes von Projektionsabbildungen für 3D-Bilddatensatz eines in einem Untersuchungsbereich (2) angeordneten Untersuchungsobjektes mit
    einer Röntgenquelle (12) und einem Röntgendetektor (13) zur Erfassung der Projektionabbildungen des Untersuchungsobjekts, und
    einer Steuereinheit (24) zum Führen der Röntgenquelle (12) entlang einer im Wesentlichen auf einer Kugeloberfläche liegenden Trajektorie (1) um das Untersuchungsobjekt zur Erfassung der Projektionsabbildungen,
    **dadurch gekennzeichnet,**
    **dass** die Röntgeneinrichtung derart ausgestaltet ist, dass die Röntgenquelle (12) zur Ermittlung des Satzes von Projektionsabbildungen die Trajektorie kontinuierlich durchlaufen kann und dass nicht alle Punkte der Trajektorie in einer gemeinsamen Ebene liegen.

12. Röntgeneinrichtung nach Anspruch 11,
    **dadurch gekennzeichnet,**
    **dass** die Röntgeneinrichtung ein C-Arm-System mit einen C-Arm (11) ist, der von einer C-Arm-Halterung (22) gehalten wird.

13. Röntgeneinrichtung nach Anspruch 12,

**dadurch gekennzeichnet,**
**dass** der C-Arm (11) und die C-Arm Halterung (22) gleichzeitig um eine C-Arm Achse (19) bzw. eine Propellerachse (14) rotierbar sind, wobei die Propellerachse (14) und die C-Arm Achse (19) zueinander orthogonal sind und sich in einem Isozentrum (18) schneiden.

14. Röntgeneinrichtung nach Anspruch 12,
**dadurch gekennzeichnet,**
**dass** die Propellerachse (14) und der C-Arm (11) im Wesentlichen in einer gemeinsamen Ebene liegen.

15. Röntgeneinrichtung nach Anspruch 12,
**dadurch gekennzeichnet,**
**dass** die Propellerachse (14) bei einer Rotation des C-Arms (11) um die C-Arm Achse (19) unverändert bleibt.

16. Röntgeneinrichtung nach Anspruch 11,
**dadurch gekennzeichnet,**
**dass** die Röntgeneinrichtung zur Erfassung von Kegelstrahlprojektionen ausgsstaltet ist.

17. Röntgeneinrichtung nach Anspruch 11,
**dadurch gekennzeichnet,**
**dass** die Drehung um die Propellerachse ein Vielfaches von 360° betragen kann.

FIG.1

FIG.2a

FIG.2b

FIG.2c

FIG.2d

FIG.3a

FIG.3b

FIG.3c

FIG.3d

FIG.4a

FIG.4b

FIG.4c

FIG.4d

FIG.5

FIG.6

FIG.7

FIG.8

| Trajektorie aus | Rotationswinkel für 1. Ausführungsbeispiel | Rotationswinkel für 2. Ausführungsbeispiel |
|---|---|---|
| Fig. 10a | $\theta_1(s) = 0$<br>$\theta_2(s) = -\pi/2 + 2\pi s$<br>$\theta_3(s) = 3/4\ (\pi/4 - \pi s/2)$ | $\theta_1(s) = \pi/2$<br>$\theta_2(s) = -\pi/2 + (1/2 - s)\ \pi/3$<br>$\theta_3(s) = -2\pi s$ |
| Fig. 11a | $\theta_1(s) = 0$<br>$\theta_2(s) = -\pi/2 + 2\pi s$<br>$\theta_3(s) = \cos[\pi s]\ \pi/12$ | $\theta_1(s) = \pi/2$<br>$\theta_2(s) = -\pi/2 + \cos[\pi s]\ \pi/10$<br>$\theta_3(s) = -2\pi s$ |
| Fig. 12a | $\theta_1(s) = 0$<br>$\theta_2(s) = -\pi/2 + 2\pi s\ 3/4$<br>$\theta_3(s) = 3/4\ (\pi/4 - \pi s/2)$ | $\theta_1(s) = \pi/2$<br>$\theta_2(s) = -\pi/2 + (3/8 - s3/4)\ \pi$<br>$\theta_3(s) = -\pi/4 - 3\pi s/2$ |
| Fig. 13a | $\theta_1(s) = 0$<br>$\theta_2(s) = \pi/2 - (135/360)\pi - (225/360)2\pi\sin^2[\pi s/2]$<br>$\theta_3(s) = \sin[2\pi s]/2$ | |
| Fig. 14a | $\theta_1(s) = 0$<br>$\theta_2(s) = -\pi/4 + 2\pi s$<br>$\theta_3(s) = -\cos[4\pi s]/4$ | $\theta_1(s) = \pi/2$<br>$\theta_2(s) = -\pi/2 - \sin[2\pi s]/2$<br>$\theta_3(s) = -2\pi s$ |
| Fig. 15a | $\theta_1(s) = 0$<br>$\theta_2(s) = 2\pi s$<br>$\theta_3(s) = -\text{sign}[s-1/2]\sin^4[2\pi s]/2$ | $\theta_1(s) = \pi/2$<br>$\theta_2(s) = -\pi/2 + \text{sign}[s-1/2]\sin^3[2\pi s]/2$<br>$\theta_3(s) = -\pi/2 - 2\pi s$ |
| Fig. 16a | $\theta_1(s) = 0$<br>$\theta_2(s) = -\pi/2 + 4\pi s$<br>$\theta_3(s) = \cos[2\pi s]/12$ | $\theta_1(s) = \pi/2$<br>$\theta_2(s) = -\pi/2 + \cos[2\pi s]\ \pi/10$<br>$\theta_3(s) = -4\pi s$ |
| Fig. 17a | $\theta_1(s) = 0$<br>$\theta_2(s) = 4\pi s$<br>$\theta_3(s) = -\text{sign}[s-1/2]\sin^4[4\pi s]/2$ | $\theta_1(s) = \pi/2$<br>$\theta_2(s) = -\pi/2 + \text{sign}[s-1/2]\sin^3[4\pi s]/2$<br>$\theta_3(s) = -\pi/2 - 4\pi s$ |
| Fig. 18a | $\theta_1(s) = 0$<br>$\theta_2(s) = \pi/2 - 3\pi/8 - 5\pi\sin^2[\pi s]/4$<br>$\theta_3(s) = \sin[4\pi s]/4$ | |
| Fig. 19a | $\theta_1(s) = 0$<br>$\theta_2(s) = -\pi/2 + 24\pi s$<br>$\theta_3(s) = \pi\cos[\pi s]/10$ | |

## FIG.9

FIG.10a

FIG.10b

FIG.11a

FIG.11b

FIG.11c

FIG.11d

FIG.11e

FIG.12a

FIG.12b

FIG.13a

FIG.13b

FIG.13c

FIG.13d

FIG.13e

FIG.14a

FIG.14b

FIG.15a

FIG.15b

FIG.16a

FIG.16b

FIG.16c

FIG.16d

FIG.16e

FIG.17a

FIG.17b

FIG.18a

FIG.18b

FIG.18c

FIG.18d

FIG.18e

FIG.19a

FIG.19b